# EUROPEAN PATENT APPLICATION

(11) **EP 4 295 857 A1**
(43) Date of publication of application: **27.12.2023**
(21) Application number: 21898636.2
(22) Date of filing: 25.11.2021
(51) Int. Cl.: A61K 38/16, A61P 43/00, A61P 11/00, G01N 33/68, C07K 14/47, C12N 15/85

(54) **PHARMACEUTICAL COMPOSITION, FOR PREVENTING OR TREATING FIBROTIC DISEASES, COMPRISING PROGRANULIN PROTEIN OR ACTIVE FRAGMENTS THEREOF AS ACTIVE INGREDIENT**

(30) Priority: 26.11.2020 KR 20200161571; 22.11.2021 KR 20210160888
(71) Applicant: The Asan Foundation, Seoul 05505 (KR); University Of Ulsan Foundation For Industry Cooperation, Ulsan 44610 (KR)
(72) Inventor: CHO, You-Sook, Seoul 07336 (KR); SHIM, Hyun Jae, Seoul 05255 (KR); PYO, Minju, Busan 49201 (KR)
(74) Representative: Dehns
(86) International application number: PCT/KR2021/017507
(87) International publication number: WO 2022/114810

(57) **Abstract**

The present invention relates to a pharmaceutical composition and the like, for preventing or treating pulmonary or airway fibrosis, comprising a progranulin (PGRN) protein or active fragments thereof as an active ingredient. The present invention is accomplished by confirming that progranulin and active fragments thereof can effectively inhibit pulmonary and airway inflammation and fibrosis. Particularly, the present inventors injected a progranulin and active fragments (PGRN-FBAC, PGRN-BACD and PGRN-CDE) thereof to a pulmonary fibrosis animal model and, as a result, confirmed reduced infiltration of inflammatory cells into the pulmonary and airway tissues and reduced fibroblast proliferation and collagen production as well as relief in inflammation-induced tissue damage. Further, the inventors induced an inflammatory response from an epithelial cell line and pulmonary fibroblasts and treated same with progranulin and active fragments thereof and, as a result, confirmed reduced levels and activities of fibronectin, p-NFκB, COL1A1, F-actin and the like which are fibrosis markers. Since said results exhibit effects of the progranulin protein and active fragments thereof in relieving pulmonary and airway inflammation and fibrosis, the progranulin protein and active fragments thereof according to the present invention are expected to be utilized as therapeutic means for various diseases induced by pulmonary and airway fibrosis.

## Description

### [Technical Field]

The present invention relates to a use of a progranulin (PGRN) protein and a fragment thereof for preventing or treating a fibrotic disease.

This application claims priority to and the benefit of Korean Patent Application No. 10-2020-0161571, filed on November 26, 2020, and Korean Patent Application No. 10-2021-0160888, filed on November 22, 2021, the disclosures of which are incorporated herein by reference in their entirety.

### [Background Art]

Fibrosis is a phenomenon in which fibrous connective tissue in an organ or tissue is excessively formed, and characterized by damaging the structure and function of tissue due to replacement of destroyed normal tissue with fibrous connective tissue. Fibrosis may be caused by persistent infections, autoimmune responses, wounds, exposure to chemicals, etc., and when the fibrosis process is not controlled in the early stage, it leads to a pathological phenomenon.

Representative chronical diseases of the respiratory system caused by fibrosis comprise pulmonary fibrosis and airway fibrosis (bronchial fibrosis). Pulmonary fibrosis is a disease that becomes the prototype of various fibrotic diseases, and a disease in which the lung hardens due to excessive accumulation of fibrous connective tissue in lung tissue, slowing expansion and relaxation and causing difficulty in breathing. To date, there is no therapeutic method for reversing or stopping the progression of pulmonary fibrosis. While, as a main therapeutic means, an immunosuppressant such as a steroid is mainly used, such an immunosuppressant reduces a patient's overall immune function, so there is a problem of a side effect in that the patient is vulnerable to other diseases. Recently, as a drug ingredient for improving this, pirfenidone is used, but it just delays the decrease in pulmonary function due to fibrosis, and fundamental disease curing is impossible. Accordingly, it is necessary to develop a therapeutic agent that can fundamentally improve and treat pulmonary fibrosis.

Recently, airway fibrosis is also of clinical interest, and is the cause of worsening the progression of a disease from a chronic bronchial inflammatory disease, e.g., asthma, to specifically airway fibrosis, and its prognosis. Asthma is a disease in which symptoms such as shortness of breath or coughing occur repetitively and paroxysmally due to bronchial inflammation and bronchoconstriction, and still has no clear therapeutic method. Particularly, it is known that irreversible respiratory obstruction caused by airway fibrosis progresses in some of patients with severe asthma. The key histopathologic finding in this patient group is fibrosis of the bronchi and/or small bronchi, and fibrosis is not improved only by controlling bronchial inflammation. In practice, recently, new biological agents developed by targeting T2 inflammation of severe asthma exhibit a high therapeutic effect on steroidresistant inflammation, but the therapeutic effect is unclear for irreversible respiratory obstruction that has progressed to airway fibrosis. Accordingly, there is an urgent demand for the development of a therapeutic agent which fundamentally improves bronchial fibrosis and can be expected to treat asthma.

### [Disclosure]

### [Technical Problem]

To solve the above problems, the present invention was completed by confirming that progranulin (PGRN) and an active fragment thereof can effectively inhibit tissue inflammation and fibrosis.

Therefore, the present invention is directed to providing a pharmaceutical composition for preventing or treating a pulmonary or airway fibrosis disease, which comprises a PGRN protein or an active fragment thereof as an active ingredient.

The present invention is also directed to providing a pharmaceutical composition for preventing or treating a pulmonary or airway fibrosis disease, which comprises a vector comprising a polynucleotide encoding a PGRN protein or an active fragment thereof, or cells comprising the vector as an active ingredient.

The present invention is also directed to providing a kit for preventing or treating a pulmonary or airway fibrosis disease, which comprises the composition.

However, technical problems to be solved in the present invention are not limited to the above-described problems, and other problems which are not described herein will be fully understood by those of ordinary skill in the art from the following descriptions.

### [Technical Solution]

The present invention provides a pharmaceutical composition for preventing or treating a pulmonary or airway fibrosis disease, which comprises a PGRN protein or an active fragment thereof as an active ingredient.

The present invention also provides a pharmaceutical composition for preventing or treating a pulmonary or airway fibrosis disease, which comprises a vector comprising a polynucleotide encoding a PGRN protein or an active fragment thereof, or cells comprising the vector as an active ingredient.

In one embodiment of the present invention, the active fragment may be, but is not limited to, one or more selected from the group consisting of the following examples:
(a) a C granulin domain of the PGRN protein;
(b) a FBAC granulin domain of the PGRN protein;
(c) a BACD granulin domain of the PGRN protein; and
(d) a CDE granulin domain of the PGRN protein.

In another embodiment of the present invention, the PGRN protein or an active fragment thereof may comprise the amino acid sequence of any one selected from the group consisting of SEQ ID NOs: 1 to 5, but the present invention is not limited thereto.

In still another embodiment of the present invention, the composition may inhibit the fibrosis or inflammation of lung or bronchial tissue, but the present invention is not limited thereto.

In yet another embodiment of the present invention, the pulmonary or airway fibrosis disease may be one or more selected from the group consisting of pulmonary fibrosis, idiopathic pulmonary fibrosis, idiopathic interstitial pneumonia, chronic obstructive pulmonary disease, asthma, airway fibrosis, chronic bronchitis, acute bronchitis, bronchiolitis, chronic obstructive airway disease, and bronchiolitis obliterans, but the present invention is not limited thereto.

In yet another embodiment of the present invention, the composition may satisfy one or more characteristics selected from the group consisting of the following characteristics, but the present invention is not limited thereto:
(i) Inhibition of tissue infiltration of inflammatory cells;
(ii) Reduction in collagen level in tissue; and
(iii) Reduction in hydroxyproline level in tissue.

In yet another embodiment of the present invention, the composition may inhibit the level or activity of one or more selected from the group consisting of fibronectin, phosphorylated NFκB, COL1A1, and F-actin, but the present invention is not limited thereto.

In yet another embodiment of the present invention, the composition may be for treatment of a patient with one or more mutants selected from the group consisting of fibronectin, NFκB, COL1A1, and F-actin, but the present invention is not limited thereto.

In yet another embodiment of the present invention, the polynucleotide encoding the PGRN protein or an active fragment thereof may comprise any one base sequence selected from the group consisting of SEQ ID NOs: 6 to 10, but the present invention is not limited thereto.

In yet another embodiment of the present invention, the vector may be linear DNA, plasmid DNA, or a recombinant viral vector, but the present invention is not limited thereto.

In addition, the present invention provides a kit for preventing or treating a pulmonary or airway fibrosis disease, which comprises the composition according to the present invention.

In addition, the present invention provides a method of preventing or treating a pulmonary or airway fibrosis disease, which comprises administering a PGRN protein or an active fragment thereof; a vector comprising a polynucleotide encoding the PGRN protein or active fragment thereof; and/or cells comprising the vector into a subject in need thereof.

In addition, the present invention provides a use of a PGRN protein or an active fragment thereof; a vector comprising a polynucleotide encoding the PGRN protein or active fragment thereof; and/or cells comprising the vector for preventing or treating a pulmonary or airway fibrosis disease.

In addition, the present invention provides a use of a PGRN protein or an active fragment thereof; a vector comprising a polynucleotide encoding the PGRN protein or active fragment thereof; and/or cells comprising the vector for preparing a drug for treating a pulmonary or airway fibrosis disease.

### [Advantageous Effects]

The present invention relates to a pharmaceutical composition for preventing or treating a pulmonary or airway fibrosis disease, which comprises a PGRN protein or an active fragment thereof as an active ingredient, and was completed by confirming that progranulin (PGRN) and an active fragment thereof can effectively inhibit pulmonary and airway inflammation and fibrosis. Particularly, as a result of injecting PGRN and an active fragment thereof (PGRN-FBAC, PGRN-BACD, or PGRN-CDE) into a pulmonary fibrosis animal model, it was confirmed that infiltration of inflammatory cells into pulmonary and bronchial tissue is reduced, fibroblast proliferation and collagen production are reduced, and tissue damage caused by inflammation is improved. In addition, as a result of treating a pulmonary epithelial cell line and pulmonary fibroblasts with PGRN and an active fragment thereof following the induction of an inflammatory response, it was confirmed that the level and activity of the fibrosis markers, fibronectin, p-NFκB, COL1A1, and F-actin, are reduced. Since the results show that the PGRN protein and active fragments thereof have an effect of improving pulmonary and airway inflammation and fibrosis, the PGRN protein according to the present invention and an active fragment thereof are expected to be used as means for treating various diseases caused by pulmonary and airway fibrosis.

### [Description of Drawings]

FIG. 1A shows the result of CBB-R250 staining after separating a fragment produced by digesting full-length human progranulin (PGRN) with neutrophil elastase (NE) through SDS-PAGE. Each line indicates PGRN digested by NE over time, and N-terminus sequencing was performed on the PGRN fragment produced under the condition of 3-hour digestion.
FIG. 1B is a cleavage map showing the gene arrangement of PGRN, PGRN-FBAC, PGRN-BACD, and PGRN-CDE to manufacture an expression vector of a PGRN protein or fragments thereof.
FIG. 1C shows the purification result through affinity chromatography after obtaining a PGRN protein or a fragment thereof from a culture of cells transformed with an expression vector overexpressing the PGRN protein or a fragment thereof.
FIG. 2A shows the process of manufacturing a bleomycin (BLM)-induced pulmonary fibrosis mouse model for 4 weeks to confirm the inhibitory effects of a PGRN protein and fragments thereof (PGRN-FBAC, PGRN-BACD, and PGRN-CDE) on fibrosis and inflammation (animal model I).
FIG. 2B shows the process of manufacturing a bleomycin-induced pulmonary fibrosis mouse model for 4 weeks to confirm the inhibitory effects of a PGRN protein and a fragment thereof (PGRN-FBAC) on fibrosis and inflammation (animal model II).
FIG. 2C shows the process of manufacturing a bleomycin-induced pulmonary fibrosis mouse model for 2 weeks to confirm the inhibitory effects of a PGRN protein and fragments thereof (PGRN-FBAC, PGRN-BACD, and PGRN-CDE) on fibrosis and inflammation (animal model III).
FIG. 3A shows the effect of reducing the number of inflammatory cells in bronchoalveolar lavage fluid according to the injection of a PGRN protein (FL) and fragments thereof (FBAC, BACD, and CDE) into a pulmonary fibrosis mouse model (animal model I).
FIG. 3B shows the effect of reducing the number of inflammatory cells in bronchoalveolar lavage fluid according to the injection of a PGRN protein and a fragment thereof (FBAC) into a pulmonary fibrosis mouse model (animal model II).
FIG. 3C shows the effect of reducing the number of inflammatory cells in bronchoalveolar lavage fluid according to the injection of a PGRN protein and fragments thereof (FBAC, BACD, and CDE) into a pulmonary fibrosis mouse model (animal model III) (PFD: mouse injected with pirfenidone).
FIG. 4A shows the result of confirming the effect of inhibiting lung infiltration of inflammatory cells by a PGRN protein and fragments thereof (FBAC, BACD, and CDE) through H&E staining of lung tissue of a pulmonary fibrosis mouse model (animal model I) (100 magnification).
FIG. 4B shows the result of confirming the effect of inhibiting lung infiltration of inflammatory cells by a PGRN protein and a fragment thereof (FBAC) through H&E staining of lung tissue of a pulmonary fibrosis mouse model (animal model II) (top, 12.5 magnification; bottom, 100 magnification).
FIG. 4C shows the result of confirming the effect of inhibiting lung infiltration of inflammatory cells by a PGRN protein and fragments thereof (FBAC, BACD, and CDE) through H&E staining of lung tissue of a pulmonary fibrosis mouse model (animal model III) (12.5 magnification; PFD, mouse injected with pirfenidone).
FIG. 4D shows the result of confirming the tissue damage improvement effect of a PGRN protein and a fragment thereof by measuring the degree of lung tissue damage in a pulmonary fibrosis mouse model (animal model III).
FIG. 5A shows the result of confirming the therapeutic effect of a PGRN protein and fragments thereof (FBAC, BACD, and CDE) on pulmonary fibrosis by performing M.T. staining on lung tissue of a pulmonary fibrosis mouse model (animal model I).
FIG. 5B shows the result of confirming the therapeutic effect of a PGRN protein and a fragment thereof (FBAC) on pulmonary fibrosis by performing M.T. staining on lung tissue of a pulmonary fibrosis mouse model (animal model II) (top, 12.5 magnification; bottom, 100 magnification).
FIG. 5C shows the result of confirming the therapeutic effect of a PGRN protein and fragments thereof (FBAC, BACD, and CDE) on pulmonary fibrosis by performing M.T. staining on lung tissue of a pulmonary fibrosis mouse model (animal model III) (12.5 magnification; PFD, mouse injected with pirfenidone).
FIG. 6 shows the result of confirming the therapeutic effect of PGRN and a fragment thereof (FBAC) on pulmonary fibrosis by measuring a hydroxyproline (Hyp) level in lung tissue of a pulmonary fibrosis mouse model (animal model II).
FIG. 7 is a graph showing a weight change caused by a PGRN protein or a fragment thereof (PGRN-FBAC, PGRN-BACD, or PGRN-CDE) in a bleomycin-induced pulmonary fibrosis mouse model (animal model III) for 2 weeks.
FIG. 8A shows the experimental process for confirming changes in a fibrosis-related marker and signaling molecule by a PGRN protein and fragments thereof (PGRN-FBAC, PGRN-BACD, and PGRN-CDE) in an A549 cell line.
FIG. 8B shows the result of confirming the levels of fibrosis markers, fibronectin (FN), and phosphorylated NFκB (p-NFκB), through western blotting after treating an A549 cell line with bleomycin; and a PGRN protein and a fragment thereof.
FIG. 9A shows the experimental process for confirming changes in fibrosis markers due to a PGRN protein and fragments thereof (PGRN-FBAC, PGRN-BACD, and PGRN-CDE) under a 3D culture condition using an MRC-5 cell line.
FIG. 9B shows the result of confirming the level of a fibrosis marker COL1A1 through western blotting after treatment with TFGβ1 (TGF); and a PGRN protein and a fragment thereof under a 3D culture condition using an MRC-5 cell line.
FIG. 9C shows the result of measuring the secretion degree of COL1A1 through ELISA after treatment with TFGβ1 (TGF); and a PGRN protein and a fragment thereof under a 3D culture condition using an MRC-5 cell line.
FIG. 9D shows the result of confirming the level of a fibrosis marker F-actin through immunofluorescence staining after treatment with TFGβ1 (TGF); and a PGRN protein and a fragment thereof under a 3D culture condition using an MRC-5 cell line.

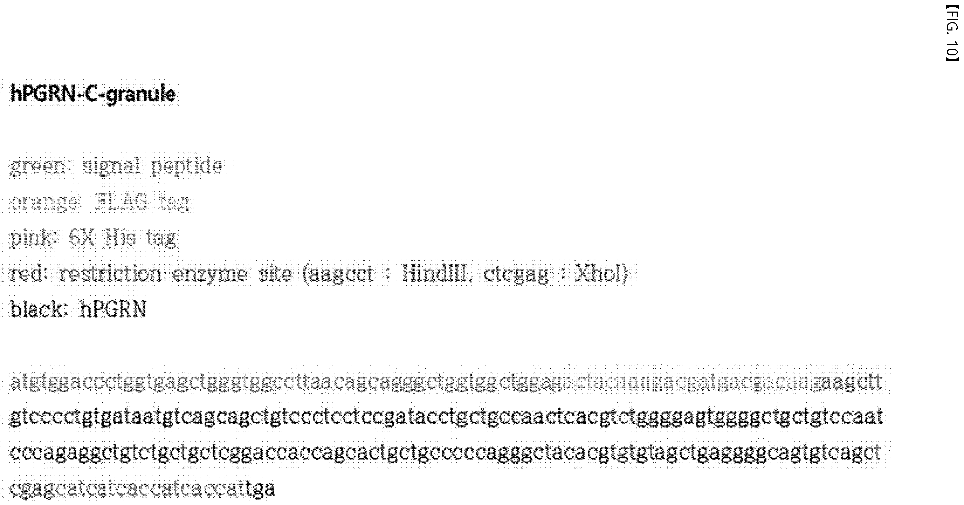

FIG. 10 shows an example of a nucleic acid sequence encoding a C granulin domain of a PGRN protein.

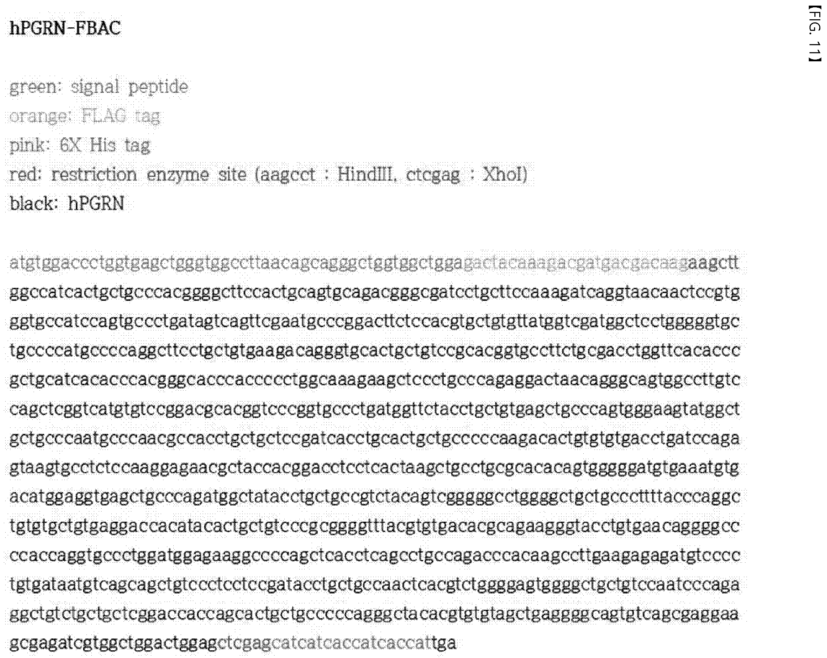

FIG. 11 shows an example of a nucleic acid sequence encoding a FBAC granulin domain of a PGRN protein.

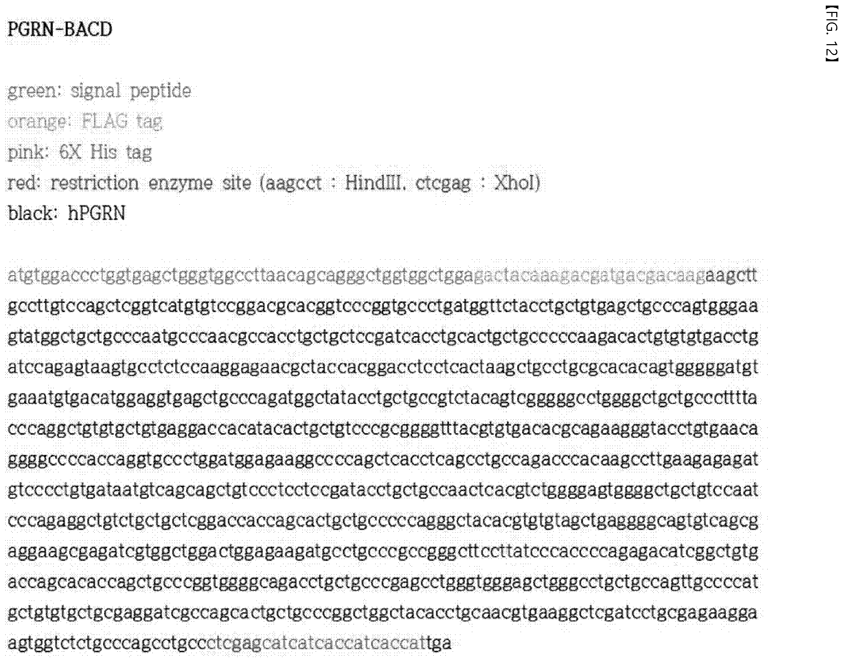

FIG. 12 shows an example of a nucleic acid sequence encoding a BACD granulin domain of a PGRN protein.

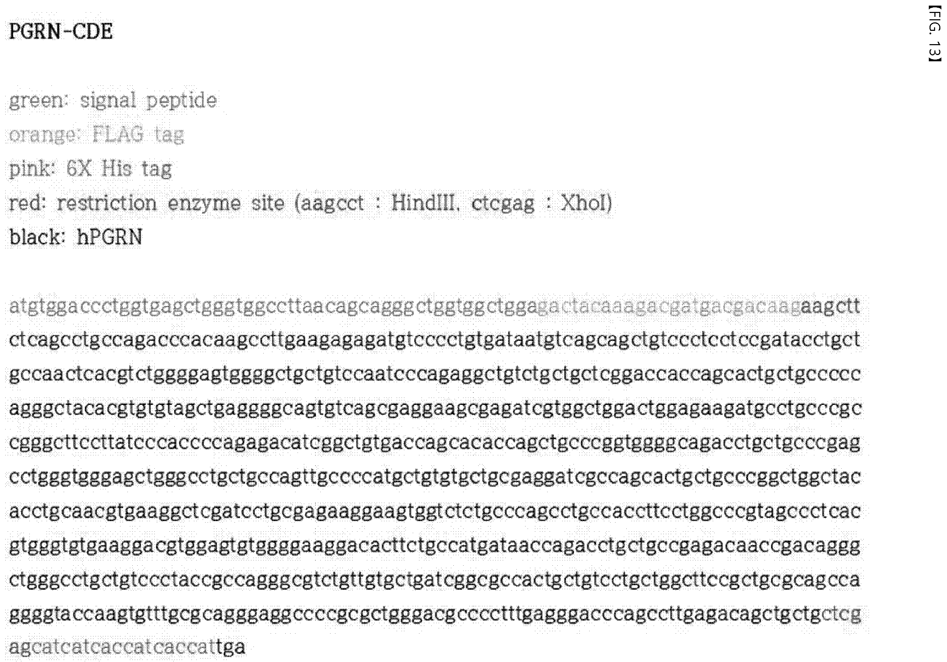

FIG. 13 shows an example of a nucleic acid sequence encoding a CDE granulin domain of a PGRN protein.
FIG. 14 shows an example of a nucleic acid sequence encoding a full-length PGRN protein.

### [Modes of the Invention]

The present invention relates to a pharmaceutical composition for preventing or treating a pulmonary or airway fibrosis disease, which comprises a progranulin (PGRN) protein or an active fragment thereof as an active ingredient, and it was completed by confirming that PGRN and an active fragment thereof can effectively inhibit pulmonary and airway inflammation and fibrosis.

Specifically, in one embodiment of the present invention, an expression vector that expresses a full-length PGRN protein according to the present invention was manufactured, and based on the PGRN protein prepared therefrom, three fragments (PGRN-FBAC, PGRN-BACD, and PGRN-CDE) were identified, and the expression vector for each fragment was manufactured (Example 1).

In another embodiment of the present invention, to verify the pulmonary and airway fibrosis inhibitory effect of a PGRN protein according to the present invention and fragments thereof, three kinds of pulmonary fibrosis mouse models (animal model I, II, and III) into which the PGRN protein or fragments thereof were injected while inducing pulmonary fibrosis with bleomycin were manufactured (Example 2).

In still another embodiment of the present invention, as a result of measuring the number of inflammatory cells by securing bronchoalveolar lavage fluid from an animal model manufactured in Example 2, it was confirmed that the number of inflammatory cells is significantly reduced in the mouse into which the PGRN protein or a fragment thereof is injected, compared with a control (Example 3).

In yet another embodiment of the present invention, as a result of performing H&E staining on lung tissue obtained from an animal model manufactured in Example 2, it was confirmed that the tissue infiltration of inflammatory cells is reduced in a mouse into which a PGRN protein or a fragment thereof is injected (Example 4).

In yet another embodiment of the present invention, as a result of performing M.T. staining on lung tissue obtained from an animal model manufactured in Example 2, it was confirmed that the proliferation of fibroblasts or the like decreases and a collagen level is reduced, and tissue damage is improved in a mouse into which a PGRN protein or a fragment thereof is injected (Example 5).

In yet another embodiment of the present invention, as a result of measuring the level of hydroxyproline, which is a major component of collagen by securing lung tissue from an animal model manufactured in Example 2, it was confirmed that the hydroxyproline level is significantly reduced in a mouse into which a PGRN protein or a fragment thereof is injected, compared with a control (Example 6).

In yet another embodiment of the present invention, as a result of confirming the time-dependent change in weight of an animal model manufactured in Example 2, it was confirmed that a decrease in weight of the mouse caused by pulmonary fibrosis is restored by the injection of a PGRN-BACD fragment (Example 7).

In yet another embodiment of the present invention, as a result of treating a pulmonary epithelial cell line with a PGRN protein and a fragment thereof, together with bleomycin, it was confirmed that the level of fibronectin, which is a fibrosis marker, is reduced, and the phosphorylation of NFκB, which is a fibrosis mediator, is reduced in the cells treated with the PGRN protein and a fragment thereof, compared with a control (Example 8).

In yet another embodiment of the present invention, as a result of treating a pulmonary fibroblast cell line with a PGRN protein and a fragment thereof, together with TGFβ1, it was confirmed that levels and activities of OL1A1 and F-actin, which are fibrosis markers, are reduced, compared to a control (Example 9).

Since the above examples support that the PGRN protein according to the present invention and active fragments thereof have excellent inflammation and fibrosis inhibitory effects in the lungs/airway, the PGRN protein according to the present invention and a fragment thereof are expected to be used in preparations for preventing or treating various types of diseases associated with pulmonary and airway fibrosis.

Hereinafter, the present invention will be described in detail.

The present invention provides a pharmaceutical composition for preventing or treating a pulmonary or airway fibrosis disease, which comprises a PGRN protein or an active fragment thereof as an active ingredient.

The "progranulin (PGRN)" used herein is a secretory protein encoded by the *GRN* gene in humans, expressed in the CNS and various types of tissue, and is highly conserved. The PGRN protein or a granulin protein produced therefrom is known to be involved in cell growth, survival, or repair. Detailed information on the PGRN protein can be confirmed in the protein database Uniprot (https://www.uniprot.org) (Accession No: P28799). The granulin protein is produced by degrading the PGRN protein. The full-length PGRN protein comprises seven full-length granulin domains (that is, granulin proteins) and one half-length granulin domain. The granulin domains are named p, G, F, B, A, C, D, and E in order from the N terminus to the C terminus of PGRN ("p" represents a half-length paragranulin domain). The molecular structure of each granulin domain is known.

The "PGRN protein or an active fragment thereof' used herein refers to a wild-type or recombinant full-length PGRN protein or a protein with substantially identical physiological activity thereto. The proteins with substantially identical physiological activity comprise the wild-type or recombinant PGRN protein as well as fragments thereof, functional equivalents thereof, and functional derivatives thereof. The "active fragment" of the PGRN protein is preferably a granulin domain of the PGRN protein. That is, the active fragment of the PGRN protein used herein may be a p, G, F, B, A, C, D, or E granulin domain, or a combination thereof. Most preferably, the active fragment of the PGRN protein in the present invention may be one or more selected from the group consisting of a C granulin domain; a FBAC granulin domain (PGRN-FBAC); a BACD granulin domain (PGRN-BACD); and a CDE granulin domain (PGRN-CDE). Throughout the specification, the "PGRN protein" is, unless specified otherwise, a full-length PGRN protein, and may be indicated as PGRN, human PGRN (hPGRN), or full-length (FN) PGRN. In addition, throughout the specification, the "fragment" comprises an "active fragment."

Preferably, the PGRN protein according to the present invention or an active fragment thereof may consist of the amino acid sequence of any one selected from the group consisting of SEQ ID NOs: 1 to 5, but the present invention is not limited thereto, and the variants of the amino acid sequence are comprised in the scope of the present invention. In other words, a polypeptide of the present invention, which consists of the amino acid sequence represented by any one of SEQ ID NOs: 1 to 5, is a concept comprising variants in which a functional equivalent of the polypeptide constituting the same, for example, a partial amino acid sequence of the polypeptide, which is modified by deletion, substitution or insertion, but acts functionally the same as the polypeptide. Specifically, a polypeptide encoding the PGRN protein or an active fragment thereof may comprise an amino acid sequence having 70% or more sequence homology, more preferably, 80% or more sequence homology, and even more preferably, 90% or more sequence homology, and most preferably, 95% or more sequence homology with the amino acid sequence represented any one of SEQ ID NOs: 1 to 5. For example, the polypeptide encoding the PGRN protein or an active fragment thereof comprises polypeptides having 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, and 100% sequence homology. The "% sequence homology" with the polypeptide is confirmed by comparing two optimally-aligned sequences with a comparative region, and a part of the polypeptide sequence in the comparative region may comprise an addition or deletion (i.e., gap) compared with a reference sequence (not having an addition or deletion) for the optimal alignment of two sequences.

More preferably, the active fragment, which is a C granulin domain, of the PGRN protein according to the present invention may comprise the amino acid sequence of SEQ ID NO: 1, or may be encoded by a polypeptide consisting of the amino acid sequence of SEQ ID NO: 1, but the present invention is not limited thereto. That is, the C granulin domain may comprise an amino acid sequence having 70% or more, more preferably, 80% or more, even more preferably 90% or more, and most preferably 95% or more sequence homology with the amino acid sequence represented by SEQ ID NO: 1. As a specific example, the C granulin domain may consist of amino acids 364 to 417 of the full-length PGRN protein consisting of the amino acid sequence of SEQ ID NO: 5, but the present invention is not limited thereto.

The active fragment, which is a FBAC granulin domain, of the PGRN protein according to the present invention may comprise the amino acid sequence of SEQ ID NO: 2, or may be encoded by a polypeptide consisting of the amino acid sequence of SEQ ID NO: 2, but the present invention is not limited thereto. That is, the FBAC granulin domain may comprise an amino acid sequence having 70% or more, more preferably, 80% or more, even more preferably 90% or more, and most preferably 95% or more sequence homology with the amino acid sequence represented by SEQ ID NO: 2. As a specific example, the FBAC granulin domain may consist of amino acids 95 to 427 of the full-length PGRN protein consisting of the amino acid sequence of SEQ ID NO: 5, but the present invention is not limited thereto.

The active fragment, which is the BACD granulin domain, of the PGRN protein according to the present invention may comprise the amino acid sequence of SEQ ID NO: 3, or may be encoded by a polypeptide consisting of the amino acid sequence of SEQ ID NO: 3, but the present invention is not limited thereto. That is, the BACD granulin domain may comprise an amino acid sequence having 70% or more, more preferably, 80% or more, even more preferably 90% or more, and most preferably 95% or more sequence homology with the amino acid sequence represented by SEQ ID NO: 3. As a specific example, the BACD granulin domain may consist of amino acids 201 to 505 of the full-length PGRN protein consisting of the amino acid sequence of SEQ ID NO: 5, but the present invention is not limited thereto.

The active fragment, which is a CDE granulin domain, of the PGRN protein according to the present invention may comprise the amino acid sequence of SEQ ID NO: 4, or may be encoded by a polypeptide consisting of the amino acid sequence of SEQ ID NO: 4, but the present invention is not limited thereto. That is, the CDE granulin domain may comprise an amino acid sequence having 70% or more, more preferably, 80% or more, even more preferably 90% or more, and most preferably 95% or more sequence homology with the amino acid sequence represented by SEQ ID NO: 4. As a specific example, the CDE granulin domain may consist of amino acids 352 to 593 of the full-length PGRN protein consisting of the amino acid sequence of SEQ ID NO: 5, but the present invention is not limited thereto.

The full-length PGRN protein according to the present invention may comprise the amino acid sequence of SEQ ID NO: 5, or may be encoded by a polypeptide consisting of the amino acid sequence of SEQ ID NO: 5, but the present invention is not limited thereto. That is, the full-length PGRN protein may comprise an amino acid sequence having 70% or more, more preferably 80% or more, even more preferably 90% or more, and most preferably 95% or more sequence homology with the amino acid sequence represented by SEQ ID NO: 5.

The "pulmonary or airway fibrosis disease" used herein comprises all diseases that can be induced by pulmonary and/or airway (that is, bronchi) fibrosis. Throughout the specification, the "fibrotic disease" may be simply denoted as "fibrosis." Moreover, the "lung" or "lung tissue" used herein refers to, unless specified otherwise, bronchi or bronchial tissue. The "fibrosis" used herein refers to a phenomenon in which part or all of an organ hardens as fibrous connective tissue is excessively formed and replaces parenchymal tissue. This is distinct from the formation of scar tissue that appears in the process of wound healing, and as the tissue hardens, it causes functional and structural abnormalities of the tissue and a corresponding organ, causing various diseases. The "pulmonary or airway fibrosis disease" used herein may be pulmonary fibrosis, idiopathic pulmonary fibrosis, idiopathic interstitial pneumonia, chronic obstructive pulmonary disease, asthma, airway fibrosis, chronic bronchitis, acute bronchitis, bronchiolitis, chronic obstructive airway disease, and/or bronchiolitis obliterans, but the present invention is not limited thereto.

The composition according to the present invention is characterized by inhibiting, improving, or treating fibrosis and/or inflammation of lung or bronchial tissue. Particularly, the composition according to the present invention may inhibit, improve, or treat irreversible respiratory obstruction accompanying severe asthma. The "severe asthma" used herein may be a condition in which an FEV1/FVC ratio is 0.7 or less, and FEV1 continuously decreases in a clinical pulmonary function test; or asthma whose symptoms are not controlled even by Stage 4 or 5 therapy in accordance with the GINA 2020 guidelines. A key histologic finding of severe asthma is fibrosis of bronchi and/or small bronchi. The bronchial fibrosis may not be improved by only inflammation control. The present inventors confirmed that the PGRN protein according to the present invention and a fragment thereof inhibit pulmonary/bronchial fibrosis through specific examples, showing that the PGRN protein and a fragment thereof improve bronchial fibrosis of severe asthma to treat irreversible respiratory obstruction.

The present inventors confirmed that when a pulmonary fibrosis animal model was treated with the PGRN protein and fragment thereof according to the present invention, the infiltration of inflammatory cells is reduced, and the levels/activities of various fibrotic and inflammatory factors are reduced, through specific examples (refer to Examples 3 to 9). Accordingly, the composition according to the present invention may satisfy one or more selected from the group consisting of the following characteristics: (i) inhibition of tissue infiltration of inflammatory cells; (ii) reduction in collagen level in tissue; and (iii) reduction in hydroxyproline level in tissue.

In addition, the composition according to the present invention may inhibit the levels or activities of one or more selected from the group consisting of fibronectin, phosphorylated NFκB (p-NFκB), COL1A1, and F-actin. Preferably, the composition may inhibit the levels or activities of one or more selected from the group consisting of fibronectin, p-NFκB, COL1A1, and F-actin of pulmonary cells or fibroblasts. Inhibiting the level of p-NFκB comprises inhibiting the phosphorylation of NFκB. All of the above proteins are major markers and mediators of fibrosis or inflammation, and the increase in their levels/activities has a positive correlation with an increase in inflammation and fibrosis.

Accordingly, the composition according to the present invention may be particularly effective in treating patients with one or more mutants selected from the group consisting of fibronectin, NFκB, COL1A1, and F-actin. The mutants comprise overactivity and/or overexpression of the inflammation and fibrosis markers.

In addition, the present invention provides a pharmaceutical composition for preventing or treating a pulmonary or airway fibrosis disease, which comprises a vector comprising a polynucleotide encoding a PGRN protein or an active fragment thereof, or cells comprising the vector as an active ingredient. That is, the present invention provides a recombinant vector comprising a polynucleotide encoding a PGRN protein or an active fragment thereof.

The "polynucleotide" used herein is an oligomer or polymer comprising two or more linked nucleotides or nucleotide derivatives, which are generally bonded to each other by a phosphodiester bond, and comprise deoxyribonucleic acid (DNA) and ribonucleic acid (RNA). A polynucleotide also comprises, for example, nucleotide derivatives, or DNA and RNA derivatives which have a "skeletal bond", such as a phosphotriester bond, a thiophosphoramidate bond, a phosphorothioate bond, a thioester bond or a peptide bond (peptide nucleic acid), other than a phosphodiester bond. A polynucleotide comprises single-stranded and/or double-stranded polynucleotides, for example, DNA and RNA, as well as derivatives of any one of RNA or DNA.

The "recombinant vector" used herein refers to a vector that can express a peptide or protein encoded by a heterogeneous nucleic acid inserted thereinto, and preferably, a vector manufactured to express a target protein (in the present invention, a PGRN protein or fragments thereof). The "vector" refers to any vehicle for the introduction and/or transfer of a base into a host cell *in vitro, ex vivo* or *in vivo,* and may be a replicon to which another DNA fragment binds and is able to bring about the replication of the fragment. The "replicon" refers to any genetic unit (e.g., a plasmid, a phage, a cosmid, a chromosome, or a virus) which functions as a self-unit for DNA replication *in vivo,* that is, is capable of replicating under its own control.

The vector according to the present invention may be linear DNA, plasmid DNA, or a recombinant viral vector, but the present invention is not limited thereto. The vector comprises, for example, a plasmid vector, a cosmid vector, a bacteriophage vector, and viral vectors such as an adenovirus vector, a lentivirus vector, a retrovirus vector, and an adeno-associated virus vector. A vector that can be used as a recombinant vector may be manufactured by manipulating a plasmid which is often used in the art (e.g., pSC101, pGV1106, pACYC177, ColE1, pKT230, pME290, pBR322, pUC8/9, pUC6, pBD9, pHC79, pIJ61, pLAFR1, pHV14, pGEX series, pET series, and pUC19), a phage (λgt4λB, λ-Charon, and M13'), or a virus (SV40). In the present invention, for exemplary examples, a pPM-C-His vector was used.

The recombinant vector of the present invention preferably comprises a promoter, which is a transcription initiation factor to which an RNA polymerase binds, an arbitrary operator sequence for regulating transcription, a sequence encoding an appropriate mRNA ribosome-binding site and a sequence regulating the termination of transcription and translation, and a terminator, and more preferably, further comprises a polyhistidine tag (an amino acid motif consisting of at least five histidine residues), a signal peptide gene, an endoplasmic reticulum retention signal peptide, a cloning site, a gene for tagging, and a marker gene for selection such as an antibioticresistant gene for selecting a transformant. In the recombinant vector, the polynucleotide sequence of each gene is operably linked to a promoter. The term "operably linked" used herein means functional bonding between a nucleotide expression regulatory sequence such as a promoter sequence and another nucleotide sequence, and thus the regulatory sequence regulates the transcription and/or translation of the other nucleotide sequence.

The recombinant vector may be constructed by using a prokaryotic cell or eukaryotic cell as a host. For example, when the vector of the present invention is an expression vector and a prokaryotic cell is used as a host, strong promoters capable of carrying out transcription (e.g., a pLλ promoter, a trp promoter, a lac promoter, a tac promoter, a T7 promoter and the like), a ribosome-binding site for initiating translation, and a transcription/translation termination sequence are generally comprised. When a eukaryotic cell is used as a host, the origin of replication of a vector, which is operated in eukaryotic cells, may comprise an f1 origin of replication, an SV40 origin of replication, a pMB 1 origin of replication, an adeno origin of replication, an AAV origin of replication, and a BBV origin of replication, but the present invention is not limited thereto. In addition, for the recombinant vector, a promoter derived from the genome of a mammalian cell (e.g., a methallothionein promoter) or a promoter derived from a mammalian virus (e.g., an adenovirus late promoter, a vaccina virus 7.5K promoter, an SV40 promoter, a cytomegalovirus promoter and a HSV kt promoter) may be used, and the recombinant vector generally has a polyadenylation sequence as a transcription termination sequence.

As the gene for tagging, representatively, an Avi tag, a Calmodulin tag, a polyglutamate tag, an E tag, a FLAG tag, an HA tag, a His tag (polyhistidine tag), a Myc tag, an S tag, a SBP tag, a IgG-Fc tag, a CTB tag, a Softag 1 tag, a Softag 3 tag, a Strep tag, a TC tag, a V5 tag, a VSV tag, or an Xpress tag may be comprised. Preferably, the vector according to the present invention may comprise a FLAG tag or a 6X His tag. More preferably, the FLAG tag may consist of a base sequence of SEQ ID NO: 12, and the 6X His tag may consist of a base sequence of SEQ ID NO: 13.

The "signal peptide" is a signal peptide located at the N-terminus of a protein and serves to induce a newly-synthesized protein to enter the endoplasmic reticulum (ER). When the signal peptide according to the present invention is a signal peptide known to one of ordinary skill in the art, its type and amino acid sequence are not limited, but it preferably consists of a base sequence of SEQ ID NO: 14.

In addition, the vector according to the present invention may further comprise a restriction site. The restriction stie is not limited to a specific type, but may be, for example, HindIII, XhoI, SmaI, or HhaI.

The vector according to the present invention may comprise a polynucleotide encoding a signal peptide; a polynucleotide encoding an FILAG tag; a polynucleotide encoding a PGRN protein or a fragment thereof; and a 6X His tag, which are sequentially linked, but the present invention is not limited to this order. In addition, the vector according to the present invention may selectively comprise a restriction site between genes.

In the present invention, a polynucleotide encoding a PGRN protein or an active fragment thereof may comprise a base sequence of any one selected from the group consisting of SEQ ID NOs: 6 to 10. Like the above-described polypeptide, the polynucleotide according to the present invention comprises a functional equivalent thereof. Therefore, the polynucleotide encoding the PGRN protein or an active fragment thereof may comprise a base sequence having 70% or more, more preferably 80% or more, even more preferably 90% or more, and most preferably 95% or more sequence homology with the base sequence of any one selected from the group consisting of SEQ ID NOs: 6 to 10.

More preferably, the active fragment, which is a C granulin domain, of the PGRN protein according to the present invention may comprise the base sequence of SEQ ID NO: 6 or may be encoded by a polynucleotide consisting of the base sequence of SEQ ID NO: 6, and may comprise a base sequence having 70% or more, more preferably 80% or more, even more preferably 90% or more, and most preferably 95% or more sequence homology with the base sequence represented by SEQ ID NO: 6.

The active fragment, which is a FBAC granulin domain, of the PGRN protein according to the present invention may comprise the base sequence of SEQ ID NO: 7 or may be encoded by a polynucleotide consisting of the base sequence of SEQ ID NO: 7, and may comprise a base sequence having 70% or more, more preferably 80% or more, even more preferably 90% or more, and most preferably 95% or more sequence homology with the base sequence represented by SEQ ID NO: 7.

The active fragment, which is a BACD granulin domain, of the PGRN protein according to the present invention may comprise the base sequence of SEQ ID NO: 8 or may be encoded by a polynucleotide consisting of the base sequence of SEQ ID NO: 8, and may comprise a base sequence having 70% or more, more preferably 80% or more, even more preferably 90% or more, and most preferably 95% or more sequence homology with the base sequence represented by SEQ ID NO: 8.

The active fragment, which is a CDE granulin domain, of the PGRN protein according to the present invention may comprise the base sequence of SEQ ID NO: 9 or may be encoded by a polynucleotide consisting of the base sequence of SEQ ID NO: 9, and may comprise a base sequence having 70% or more, more preferably 80% or more, even more preferably 90% or more, and most preferably 95% or more sequence homology with the base sequence represented by SEQ ID NO: 9.

The full-length PGRN protein according to the present invention may comprise the base sequence of SEQ ID NO: 10 or may be encoded by a polynucleotide consisting of the base sequence of SEQ ID NO: 10, and may comprise a base sequence having 70% or more, more preferably 80% or more, even more preferably 90% or more, and most preferably 95% or more sequence homology with the base sequence represented by SEQ ID NO: 10.

In the present invention, the cell comprising a vector comprising a polynucleotide encoding a PGRN protein or an active fragment thereof means a cell transformed by the vector. The "transformation" used herein is the generic term for changes in the genetic properties of an organism by introduced DNA, and the "transgenic organism" means a cell or organism prepared by introducing a foreign gene, and preferably, a cell transformed by a recombinant expression vector of the present invention by a molecular genetic method. The transformant may be prepared by a method such as transformation, transfection, Agrobacterium-mediated transformation, particle gun bombardment, sonication, electroporation, and polyethylene glycol (PEG)-mediated transformation, which is not limited as long as it is a method can inject the vector of the present invention.

The content of the PGRN protein or a fragment thereof; a vector comprising a polynucleotide encoding the same; or cells comprising the vector in the composition of the present invention can be properly regulated according to symptoms of the disease, the progression of the symptoms, and the condition of a patient, and may be, for example, 0.0001 to 99.9 wt%, or 0.001 to 50 wt% based on the total weight of the composition, but the present invention is not limited thereto. The content ratio is a value obtained based on a dry content from which a solvent is removed.

The pharmaceutical composition according to the present invention may further comprise an appropriate carrier, excipient and diluent, which are conventionally used to prepare a pharmaceutical composition. The excipient may be, for example, one or more selected from the group consisting of a diluent, a binder, a disintegrant, a lubricant, an adsorbent, a humectant, a film-coating material, and a controlled-release additive.

The pharmaceutical composition according to the present invention may be used after being formulated in the form of a powder, a granule, a sustained-release granule, an enteric granule, a liquid, an ophthalmic solution, an elixir, an emulsion, a suspension, a spirit, a troche, aromatic water, a lemonade, a tablet, a sustained-release tablet, an enteric tablet, a sublingual tablet, a hard capsule, a soft capsule, a sustained-release capsule, an enteric capsule, a pill, a tincture, a soft extract, a dry extract, a fluid extract, an injection, a capsule, a perfusate, a plaster, a lotion, a paste, a spray, an inhalant, a patch, a sterile injection, or a preparation for external use such as an aerosol according to a conventional method, and the preparation for external use may be formulated in the form of a cream, a gel, a patch, a spray, an ointment, a plaster, a lotion, a liniment, a paste or a cataplasma.

The carrier, excipient and diluent which may be comprised in the pharmaceutical composition according to the present invention may comprise lactose, dextrose, sucrose, an oligosaccharide, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, and mineral oil.

The pharmaceutical composition according to the present invention may be formulated with a diluent or an excipient such as a filler, a thickening agent, a binder, a wetting agent, a disintegrant, and a surfactant, which are commonly used.

The pharmaceutical composition according to the present invention is administered in a pharmaceutically effective amount. In the present invention, the "pharmaceutically effective amount" used herein refers to an amount sufficient for treating a disease at a reasonable benefit/risk ratio applicable for medical treatment, and an effective dosage may be determined by parameters comprising a type of a patient's disease, severity, drug activity, sensitivity to a drug, administration time, an administration route and an excretion rate, the duration of treatment and drugs simultaneously used, and other parameters well known in the medical field.

The pharmaceutical composition according to the present invention may be administered separately or in combination with other therapeutic agents, and may be sequentially or simultaneously administered with a conventional therapeutic agent, or administered in a single or multiple dose(s). In consideration of all of the above-mentioned parameters, it is important to achieve the maximum effect at the minimum dose without a side effect, and such a dose may be easily determined by one of ordinary skill in the art.

The pharmaceutical composition of the present invention may be administered into a subject via various routes. All administration routes may be expected, and the pharmaceutical composition of the present invention may be administered by, for example, oral administration, subcutaneous injection, intraperitoneal administration, intravenous, intramuscular or intrathecal injection, sublingual administration, buccal administration, rectal insertion, vaginal insertion, ocular administration, ear administration, nasal administration, inhalation, spraying through the mouth or nose, skin administration, or transdermal administration.

The pharmaceutical composition of the present invention is determined by the type of drug, which is an active ingredient, in addition to several related factors such as a disease to be treated, an administration route, a patient's age, sex, or weight, and the severity of the disease.

The term "subject" used herein refers to a target in need of treatment of a disease, and more specifically, a mammal such as a human or a non-human primate, a mouse, a rat, a dog, a cat, a horse, or a cow.

The "administration" used herein refers to the provision of the composition of the present invention to a subject by any appropriate method.

The "prevention" used herein refers to all actions that inhibit or delay the occurrence of a target disease, the "treatment" refers to all actions involved in alleviating or beneficially changing symptoms of a target disease and a metabolic disorder caused thereby by the administration of the pharmaceutical composition according to the present invention, and the "improvement" refers to all actions involved in reducing parameters associated with a target disease, for example, the severity of symptoms, by the administration of the composition according to the present invention.

In addition, the present invention provides a kit for preventing or treating a pulmonary or airway fibrosis disease, which comprises a PGRN protein or a fragment thereof; a vector comprising a polynucleotide encoding the same; and/or a cell comprising the vector.

The kit according to the present invention is not limited to a specific form as long as it is for preventing or treating a pulmonary or airway fibrosis disease, and may comprise a component/apparatus for increasing the expression or activity of a PGRN protein according to the present invention or a fragment thereof, or an arbitrary component and apparatus for the expression or transformation of the vector.

In addition, the present invention comprises a food composition for preventing or improving a pulmonary or airway fibrosis disease, which comprises a PGRN protein or a fragment thereof; a vector comprising a polynucleotide encoding the same; and/or a cell comprising the vector as an active ingredient. The food composition comprises a health functional food composition.

When the PGRN protein or a fragment thereof; a vector comprising a polynucleotide encoding the same; and/or a cell comprising the vector is(are) used as a food additive, the components may be added alone or used together with another food or food ingredient, and may be appropriately used according to a conventional method. The mixing amount of the active ingredient may be properly determined according to the purpose of use (prevention, health, or therapeutic treatment). Generally, in the production of a food or beverage, the PGRN protein or a fragment thereof; a vector comprising a polynucleotide encoding the same; and/or a cell comprising the vector may be added at 15 wt% or less, or 10 wt% or less based on a raw material. However, in the case of long-term intake for health and hygiene or health control, the amount may be less than the above range, and since there is no problem in terms of safety, the active ingredient may be used in an amount exceeding the above range.

There is no particular limitation on the type of food. Examples of food to which the material can be added comprise meat, sausages, bread, chocolates, candies, snacks, confectioneries, pizza, ramen, other noodles, gums, dairy products comprising ice cream, various kinds of soup, beverages, tea, drinks, alcohol beverages, and vitamin complexes, and comprises all types of health functional food in the usual sense.

The health beverage composition according to the present invention may comprise various flavoring agents or natural carbohydrates like a conventional beverage. Examples of the above-mentioned natural carbohydrates comprise conventional sugars, for example, monosaccharides such as glucose, fructose, etc.; disaccharides such as maltose, sucrose, etc.; and polysaccharides such as dextrin, cyclodextrin, etc., and sugar alcohols such as xylitol, sorbitol, erythritol, etc. As the sweeteners, natural sweeteners [thaumatin, stevia extract (e.g., rebaudioside A, glycyrrhizin, etc.)] and synthetic sweeteners (saccharin, aspartame, etc.) may be used. Generally, the proportion of the natural carbohydrate is approximately 0.01 to 0.20 g, or approximately 0.04 to 0.10 g per 100 mL of the composition of the present invention.

In addition, the composition of the present invention may comprise various nutrients, vitamins, electrolytes, flavoring agents, pectic acid and a salt thereof, alginic acid and a salt thereof, organic acids, protective colloidal thickening agents, pH adjustors, stabilizers, preservatives, glycerin, alcohols, or carbonizing agents used in carbonated beverages. Other than these, the composition of the present invention may comprise fruit pulp for producing natural fruit juices, fruit drinks and vegetable drinks. Such an ingredient may be used independently or in combination. The proportion of such an additive is not critical, but is generally selected in the range of 0.01 to 0.20 parts by weight per 100 parts by weight of the composition of the present invention.

Hereinafter, to help in understanding the present invention, exemplary examples will be suggested. However, the following examples are merely provided to understand the present invention more easily, and not to limit the present invention.

### [Examples]

### Example 1. Manufacture of expression vector expressing progranulin protein and fragment thereof

To confirm the inflammation and fibrosis inhibitory effect of a progranulin (PGRN) protein and a fragment thereof in a pulmonary fibrosis-induced animal model, an expression vector capable of expressing the proteins was manufactured.

First, to obtain a fragment of full-length human PGRN (hPGRN), full-length PGRN was mixed with active neutrophil elastase (NE; R&D Systems) and then digested. Specifically, NE and cathepsin C (R&D Systems) were mixed in an activation buffer (50 mM MES pH 5.5, 50 mM NaCl), and reacted at 37 °C for 3 hours to activate the neutrophil elastase (NE). Subsequently, the full-length hPGRN (R&D Systems) and the activated NE were mixed with a digestion buffer (50 mM Tris-HCl pH 7.4, 1M NaCl, 0.05% Brij-35), and reacted at 37 °C for 12 hours to digest hPGRN.

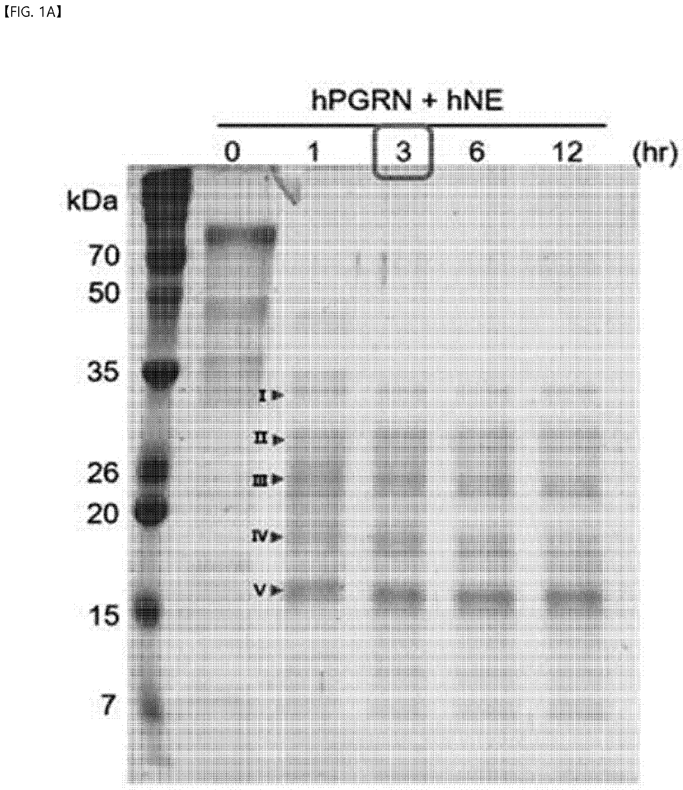

The generated fragments were observed by SDS-PAGE electrophoresis and CBB-R250 staining (FIG. 1A). The fragments on SDS-PAGE were transferred to a PVDF membrane (Pore size: 0.2 µm) and stained by CBB-R250, the PGRN fragments were cut out, and a digestion site of the PGRN cut by NE was estimated through N-terminus sequencing (Edman analysis; Core Facility, Tufts University). Based on the estimation, PGRN fragments that can be produced by NE were predicted, resulting in identification of a total of three types of fragments (PGRN-FBAC, PGRN-BACD, and PGRN-CDE).

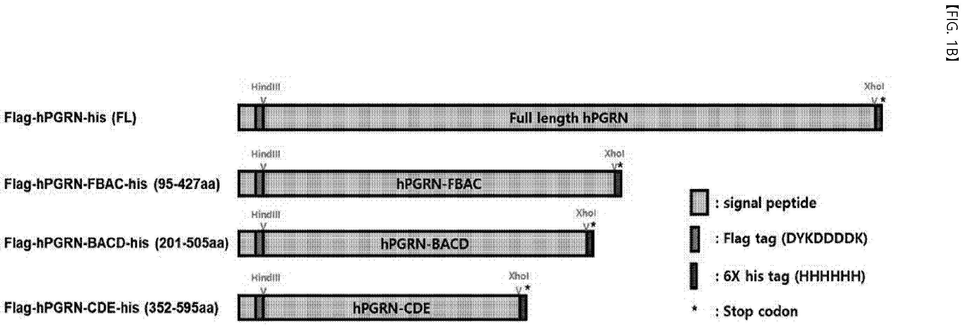

Subsequently, expression vectors which can express full-length PGRN and the three types of fragments thereof in mammalian cells were manufactured (FIG. 1B). Specifically, the full-length sequence of a hPGRN gene was inserted into a pPM-C-his vector (SEQ ID NO: 11; ABM), the signal peptide sequence, FLAG-tag, and the Hind III restriction enzyme sequence were sequentially inserted upstream of PGRN using insertion mutagenesis, and the XhoI restriction enzyme sequence and the 6X his tag sequence were sequentially inserted downstream of the PGRN sequence, thereby preparing a full-length PGRN expression vector ("pPM-flag-hPGRN full length-his expression vector").

Based on the full-length PGRN expression vector, primers of the PGRN-FBAC, PGRN-BACD, and PGRN-CDE sequences were manufactured to amplify each fragment through PCR, and then the PCR fragments were digested with HindIII (NEB) and XhoI (NEB). The pPM-flag-hPGRN full length-his expression vector was also digested with HindIII and XhoI, and the digested PCR product and digested vector were ligated using a T4 DNA ligase (Invitrogen). The ligated plasmid was transformed into XL2-BLUE to acquire a single colony and incubated in an LB (Amp) medium at 37 °C for 18 hours, and then the plasmid was extracted from proliferated strains using a DNA extraction kit (Promega) according to the manufacturer's protocol. Subsequently, each expression vector was transfected into a HEK293 cell line using a Lipofectamine 3000 reagent (ThermoFisher Scientific), and from day 2, a stable cell line was selected by treatment with neomycin.

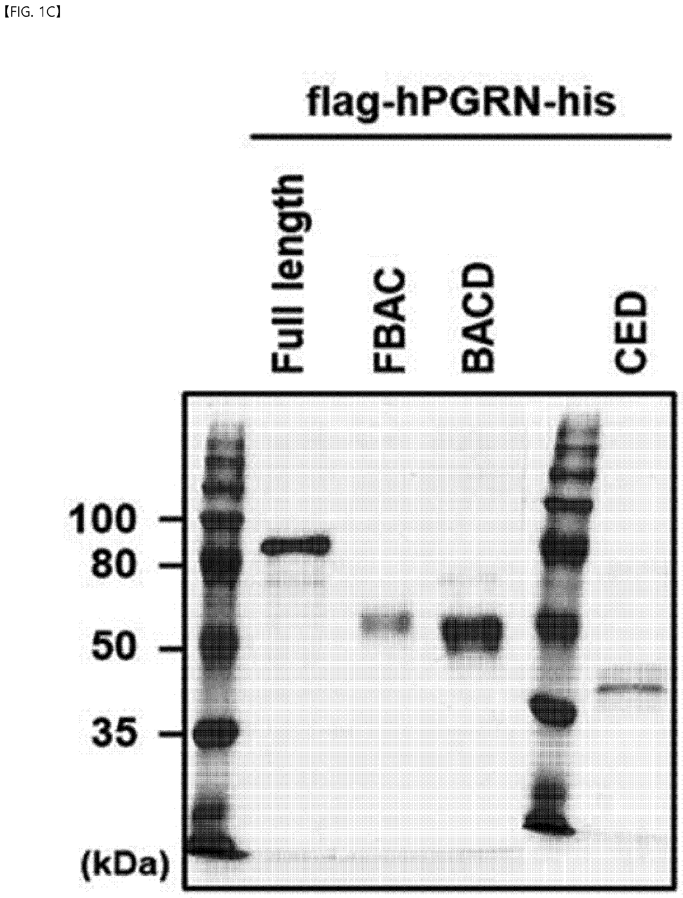

A culture obtained by culturing a cell line overexpressing each of the full-length PGRN or a fragment thereof in a serum-free medium for 4 days was concentrated in an Amicon tube and purified by affinity chromatography using FLAG-tag (FIG. 1C). First, the concentrated culture was flowed through a poly-prep mini column filled with an anti-flag M2 affinity gel (Sigma-Aldrich) five times, and a TBS (50 mM Tris-HCl (pH 7.4), 150 mM NaCl) buffer was flowed therethrough to remove a protein which non-specifically binds to the gel. Afterward, proteins specifically binding to the gel were extracted with a Flag peptide (Sigma-Aldrich). The extracted protein fraction was dialyzed with PBS for 24 hours to be treated in a cell or mouse, and then the protein was quantified by a BCA method and used in a subsequent experiment.

### Example 2. Manufacture of mouse model for verifying fibrosis inhibitory efficiency of PGRN protein and fragment thereof

Mouse models for verifying the fibrosis inhibitory effect of full-length PGRN and three types of fragments thereof were manufactured (animal model I). Particularly, for PGRN-FBAC, to further verify the effect, an additional mouse model was manufactured to perform an experiment (animal model II), and an additional mouse model was manufactured to verify the fibrosis inhibitory effect of full-length PGRN and a fragment thereof in a 2-week model rather than a 4-week model (animal model III).

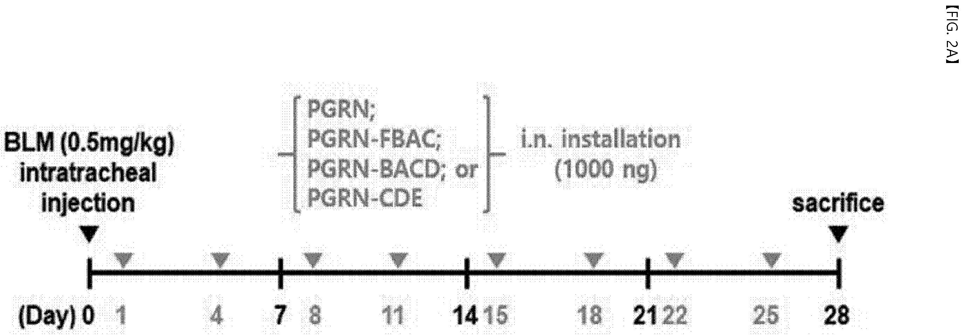

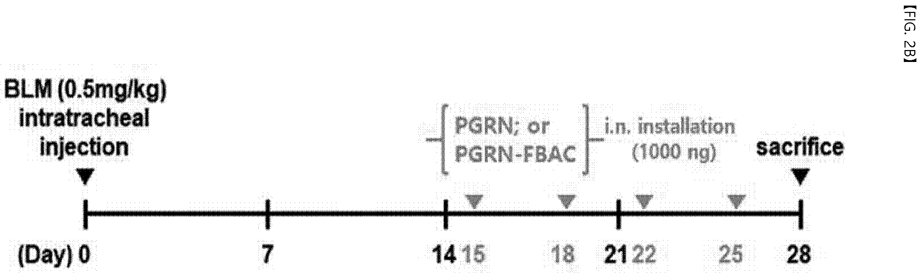

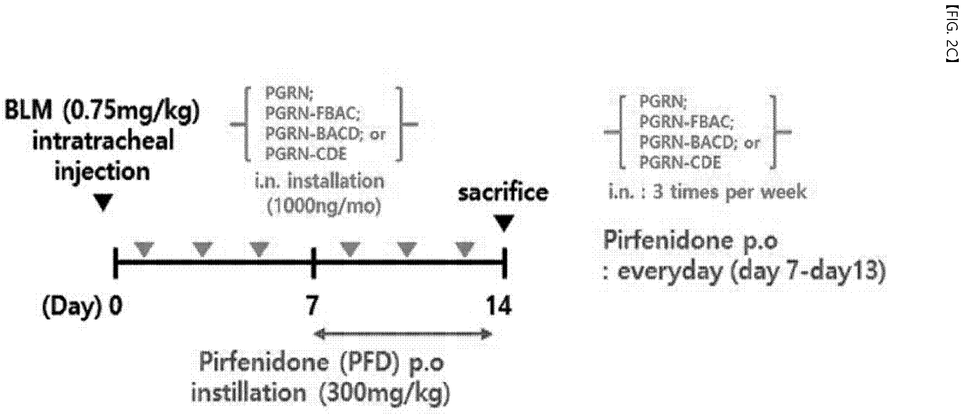

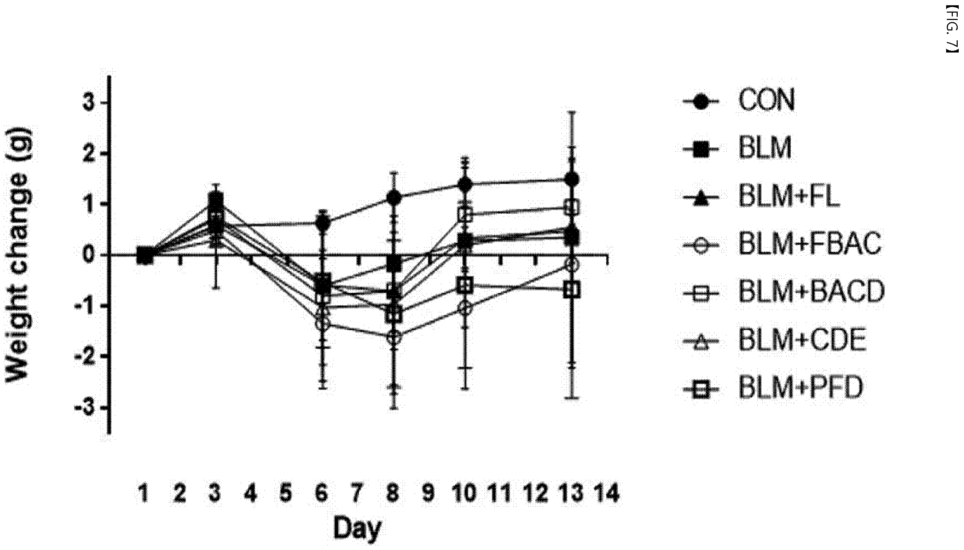

Pulmonary fibrosis of the animal models was induced using bleomycin. Specifically, for the animal model I, bleomycin was injected into the airway of 7 to 10-week-old C57BL/6 mice using a catheter, and the mice were observed for 4 weeks. A PGRN, PGRN-FBAC, PGRN-BACD, or PGRN-CDE fragment was injected into the nasal cavity twice a week for 4 weeks for a total of 8 times (FIG. 2A). For the animal model II, as described above, bleomycin was injected into the airway of 7 to 10-week-old C57BL/6 mice using a catheter, and the mice were observed for 4 weeks. A PGRN or PGRN-FBAC fragment was injected into the nasal cavity twice a week at week 3 and week 4 for a total of 4 times (FIG. 2B). For the animal model III, as described above, bleomycin was injected into the airway of 7 to 10-week-old C57BL/6 mice using a catheter, and the mice were observed for 2 weeks. A PGRN, PGRN-FBAC, PGRN-BACD, or PGRN-CDE fragment was injected into the nasal cavity three times a week for 2 weeks for a total of 6 times. In addition, to compare the pulmonary fibrosis inhibitory effect of PGRN and a fragment thereof with pirfenidone (PFD), which is a drug currently used as an agent for treating pulmonary fibrosis, from day 7 to day 13 after bleomycin injection, PFD was orally administered daily (FIG. 2C). In addition, until day 14 after bleomycin injection, weights were measured to observe a change in weight of mice (FIG. 7).

At week 4 (day 28; animal model I, II) or week 2 (day 14; animal model III) after establishment of bleomycin-induced pulmonary fibrosis models, the mice were anesthetized intraperitoneally, the chest was incised, cardiac blood collection was performed using a 26-gauge syringe to collect blood samples at the same time as euthanasia. In addition, a catheter was inserted into the airway, and PBS was treated at 1 ml twice, thereby obtaining bronchoalveolar lavage fluid, which was stored at - 80 °C for subsequent cytokine analysis.

In addition, after reperfusion using PBS, lung tissue comprising bronchial tissue was obtained. In the lung tissue, the left lung was prepared as a slide for subsequent histopathology examination. Specifically, after fixing in 10% formalin for one day, the tissue was embedded using paraffin melted at a temperature of 61 °C or less, and the paraffin section was sliced to a thickness of 4 µm using a microtome, thereby preparing a slide for H&E staining or M.T staining. Tissues other than the left lung were stored at -80 °C for protein and RNA extraction.

### Example 3. Confirmation of inflammation inhibitory effect of PGRN protein and fragment thereof through inflammatory cell analysis

In pulmonary fibrosis diseases such as chronic obstructive pulmonary disease, asthma, etc., inflammatory cells such as neutrophils, macrophages, and lymphocytes invade the lungs and bronchial tubes, causing inflammation. Accordingly, the effects of the PGRN protein and fragments thereof according to the present invention on inflammatory cells in the lungs were observed to confirm their therapeutic effects on the lungs and airway. Since bronchoalveolar lavage fluid (BALF) comprises cells derived from the bronchi, precise information about the conditions of the lungs and bronchial tubes is provided. Accordingly, bronchoalveolar lavage fluid was obtained from each of the bleomycin-induced pulmonary fibrosis models manufactured in Example 2 (animal models I, II, and III), the degrees of inflammation in the lungs and airway were confirmed by measuring the total number of inflammatory cells in the lavage fluid using a hymocytometer.

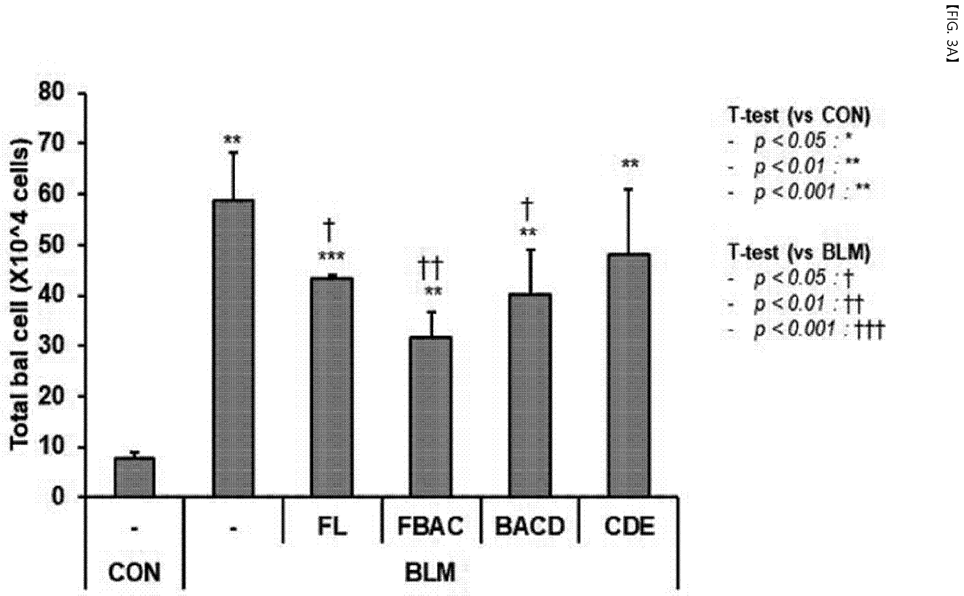

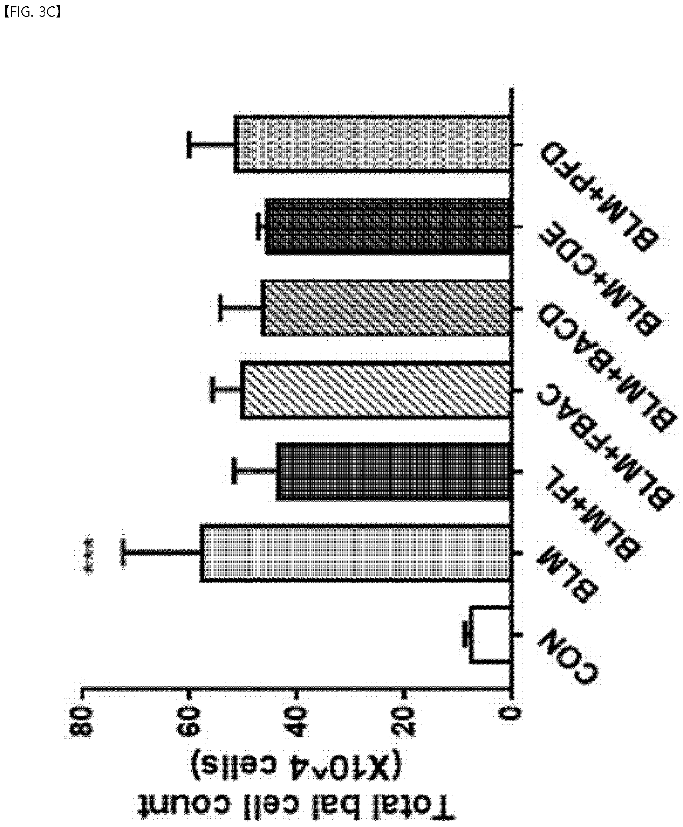

First, in the case of animal model I, as shown in FIG. 3A, when pulmonary fibrosis is induced, in a group treated with bleomycin alone (BLM), the number of inflammatory cells in the bronchoalveolar lavage fluid significantly increased compared to a control (CON). However, in a full-length PGRN protein (FL)-administered mouse, the number of inflammatory cells significantly decreased, and in a group treated with a fragment (PGRN-FBAC, PGRN-BACD, or PGRN-CDE) of the PGRN protein, it can also be found that the number of inflammatory cells in the bronchoalveolar lavage fluid remarkably decreased. Particularly, in the PGRN-FBAC-treated mice, the level of inflammatory cells was halved compared to the group treated with bleomycin alone.

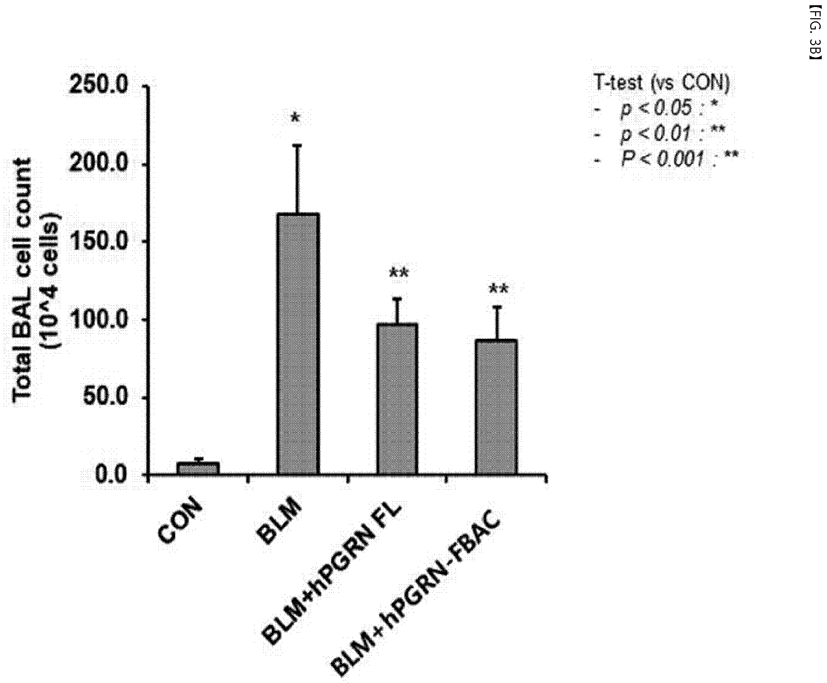

The result for animal model II is shown in FIG. 3B. Similar to the above result, as compared to the control, the number of inflammatory cells in the bronchoalveolar lavage fluid greatly increased in the group treated with bleomycin alone, but significantly decreased in the full-length PGRN protein-treated group and decreased in the PGRN-FBAC-treated group even more.

Likewise in the case of animal model III, the number of inflammatory cells in the bronchoalveolar lavage fluid was increased by bleomycin treatment, but the number of inflammatory cells decreased in the mice treated with the full-length PGRN protein, PGRN-FBAC, PGRN-BACD, or PGRN-CDE, demonstrating that the inflammation inhibitory effects caused by the PGRN protein and a fragment thereof are comparable to that of pirfenidone (PFD), which is the existing therapeutic agent for pulmonary fibrosis.

The above results show that the PGRN protein according to the present invention and fragments thereof have an effect of reducing inflammatory cells in the lungs and bronchi, thereby improving inflammation and fibrosis.

### Example 4. Confirmation of inflammation and tissue damage improvement effect of PGRN protein and fragment thereof through H&E staining

Hematoxylin and eosin (H&E) staining is an experimental method for observing the number and morphology of cells by staining the nucleus and cytoplasm of the cells. In the lungs where inflammation or fibrosis has occurred, a large amount of inflammatory cells are introduced and localized around the blood vessels and airway, so strong staining appears around the blood vessels and airway during H&E staining. Accordingly, to verify the therapeutic effect of the PGRN protein and fragments thereof on pulmonary and airway fibrosis, H&E staining was performed on the lung tissue of a pulmonary fibrosis animal model.

Specifically, each of the tissue slides prepared in Example 2 was immersed in xylene three times for 5 minutes each, in 100% ethanol for 5 minutes, and in 50% ethanol for 5 minutes. To stain a cell nucleus in tissue cleared by xylene, the cells were stained with hematoxylin for 1 minute and 40 seconds, immersed in 0.3% HCl/ethanol once or twice, and washed with running distilled water. Afterward, the slides were immersed in 0.3% ammonia twice or three times, washed with running distilled water, and immersed in eosin for 10 to 20 seconds for counter staining. After completing the staining, the slides were immersed sequentially in 50% ethanol and 100% ethanol three times and in xylene three times for 3 minutes each, and then dried. After dropping a mounting solution, the slide was covered with a cover slide to complete a tissue prep. The stained tissue was observed using an optical microscope.

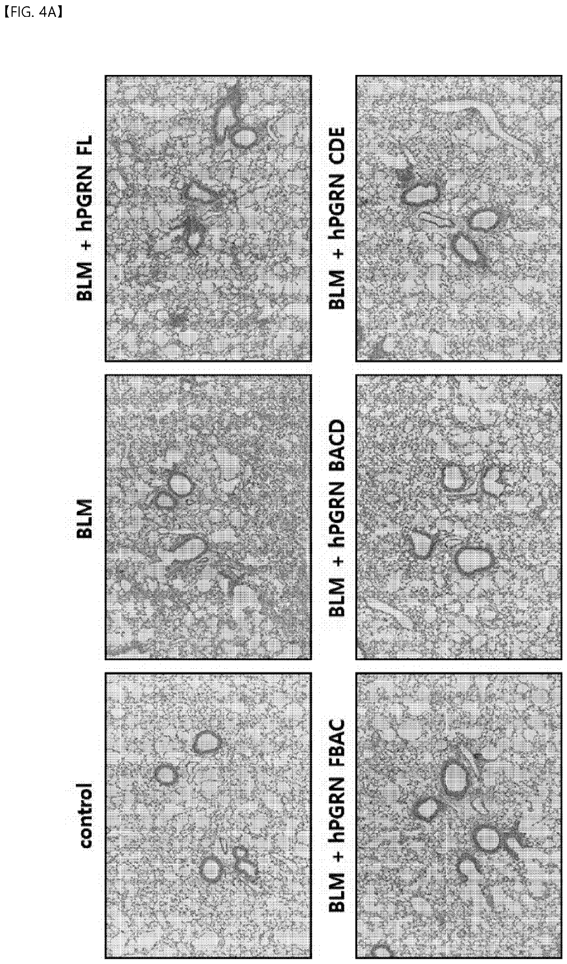

In the case of animal model I (FIG. 4A), in a group treated with bleomycin alone, it can be confirmed that, as a large number of inflammatory cells were infiltrated around the blood vessels and airway due to fibrosis occurring in lung tissue due to bleomycin, the cells were intensively stained with hematoxylin and eosin compared to a control. On the other hand, it can be seen that, in mice treated with the full-length PGRN protein or a fragment thereof (PGRN-FBAC, PGRN-BACD, or PGRN-CDE), the intensity of H&E staining was relatively reduced, showing that the infiltration of inflammatory cells into lung tissue was reduced.

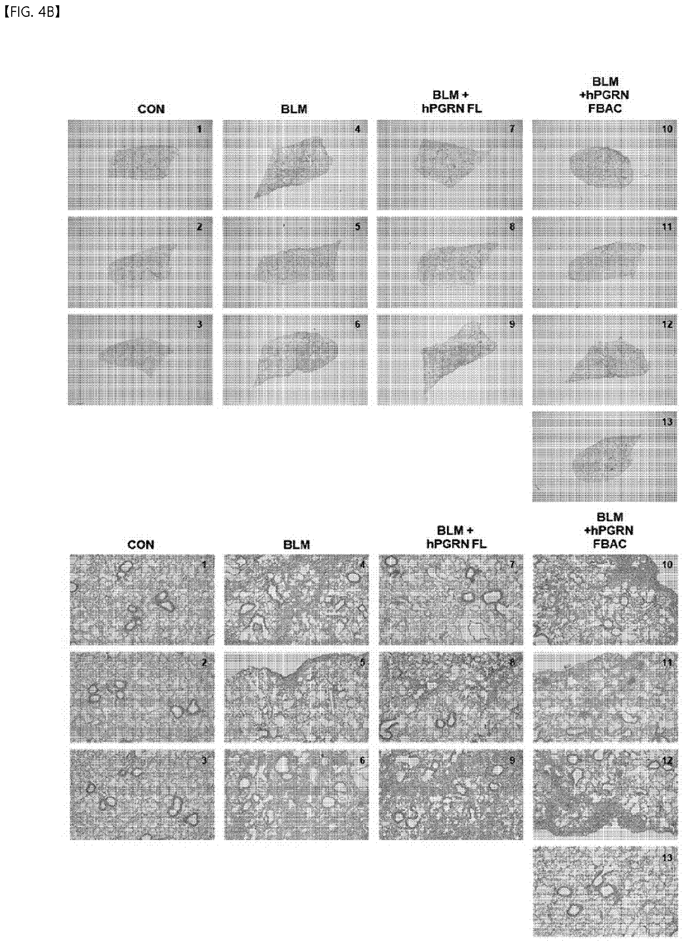

Likewise, for animal model II (FIG. 4B), in a group treated with bleomycin alone, compared to a control, H&E were intensively stained around the blood vessels and airway of the lung tissue, confirming that the infiltration of inflammatory cells increases. On the other hand, as the lung tissue of the mice into which the full-length PGRN protein or PGRN-FBAC was injected was less stained with H&E than the group treated with bleomycin alone, it can be confirmed that the infiltration of inflammatory cells is relatively inhibited.

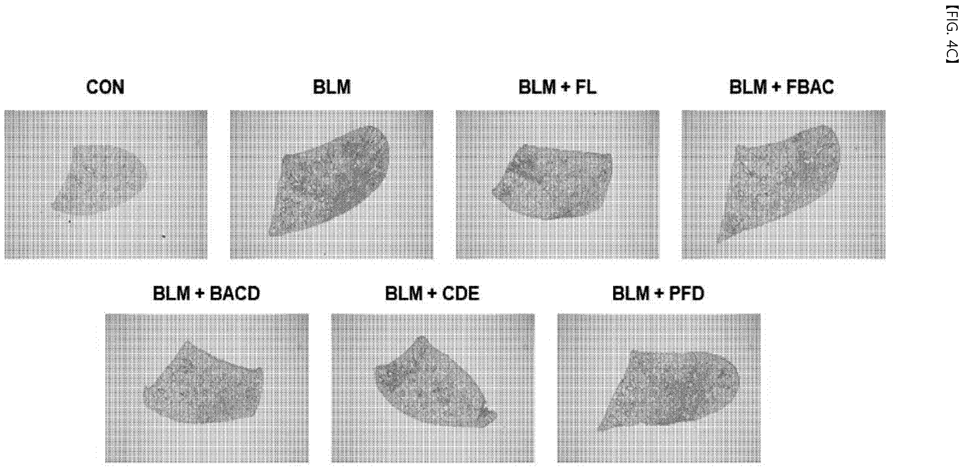

In the case of animal model III (FIG. 4C), it was confirmed that, in the mice treated with bleomycin alone, compared to the control, the infiltration of inflammatory cells into lung tissue was higher, but the degree of H&E staining was relatively lower by treating the full-length PGRN protein or a fragment thereof (PGRN-FBAC, PGRN-BACD, or PGRN-CDE), and the intensity of staining decreased compared to a pirfenidone-administered mouse. This suggests that the PGRN protein according to the present invention and fragments thereof exhibit better pulmonary inflammation inhibitory effects than pirfenidone.

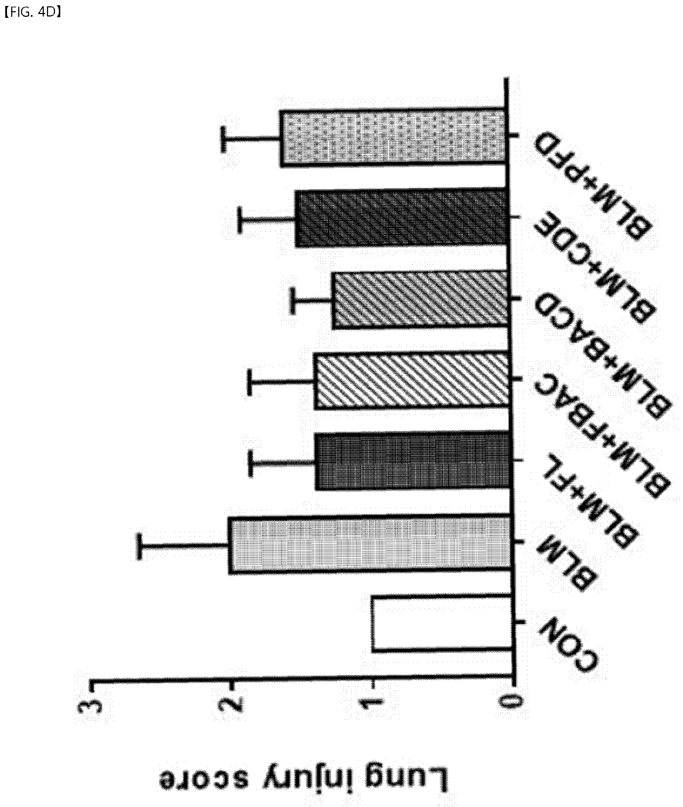

In addition, the degrees of lung tissue damage in animal model III were further compared according to whether or not the PGRN protein and fragments thereof were treated. The degrees of damage of lung tissue were scored according to the following criteria: 1: normal, 2: minor fibrosis, 3: moderate fibrosis, 4: slightly severe fibrosis, and 5: very severe fibrosis. As a result, it was confirmed that, compared to the control, the lung tissue was severely damaged in the mice treated with bleomycin alone, but the degree of damage of lung tissue remarkably decreased in the mice into which the PGRN protein or a fragment thereof was injected (FIG. 4D). Particularly, the lung tissue damage inhibitory effects of the PGRN protein and a fragment thereof are comparable to that of pirfenidone.

The above results show that the PGRN protein according to the present invention and fragments thereof cannot only inhibit the infiltration of inflammatory cells into lung tissue but also can improve tissue damage, and thus can treat pulmonary and airway fibrosis diseases.

### Example 5. Confirmation of fibrosis inhibitory effects of PGRN protein and fragment thereof through M.T. staining

Masson's Trichrome (M.T.) staining is staining for evaluating the amount of fibrous connective tissue and is used to confirm the degree of tissue fibrosis. Specifically, during M.T. staining, cell nuclei are stained black, muscle tissue and cytoplasm are stained red, and collagen fibers are stained blue. Tissue where more fibrosis has occurred is more intensively stained because the extracellular matrix comprising collagen increases along with cells such as fibroblasts and myofibroblasts. Accordingly, to confirm the therapeutic effects of the PGRN protein and fragments thereof on pulmonary and airway fibrosis, M.T. staining was performed on the lung tissue of pulmonary fibrosis animal models (animal models I, II, and III).

Specifically, each of the tissue slides prepared in Example 2 was immersed in xylene twice for 5 minutes each, in 100% ethanol twice for 3 minutes each, and in 95% ethanol for 3 minutes, and then washed with distilled water. Subsequently, tissue was fixed with a Bouin's Fixative solution (Polysciences) for 15 minutes, and washed with distilled water for 5 minutes to remove picric acid. To stain a cell nucleus, the tissue was stained using a Weigert's Iron Hematoxylin kit (Polysciences) for 10 minutes, and washed with running distilled water for 5 minutes. To stain muscle tissue and cytoplasm, the tissue was stained with Biebrich Scarlet-Acid Fuchsin (Polysciences) for 5 minutes, and washed with distilled water three times. Finally, to stain collagen, the tissue was pretreated with phosphotungstic/phosphomolybdic acid (Polysciences) for 10 minutes, the slide was dried, and then the tissue was stained with aniline blue (Polysciences) for 10 minutes and washed with distilled water twice. The stained tissue was immersed in 1% acetic acid for 1 minute and then washed with distilled water once, followed by sequential immersion in 95% ethanol 100% ethanol, and xylene for 1.5 minutes, and drying the slide. After dropping a mounting solution, the slide was covered with a cover slide, thereby manufacturing a tissue prep. The stained tissue was observed using an optical microscope.

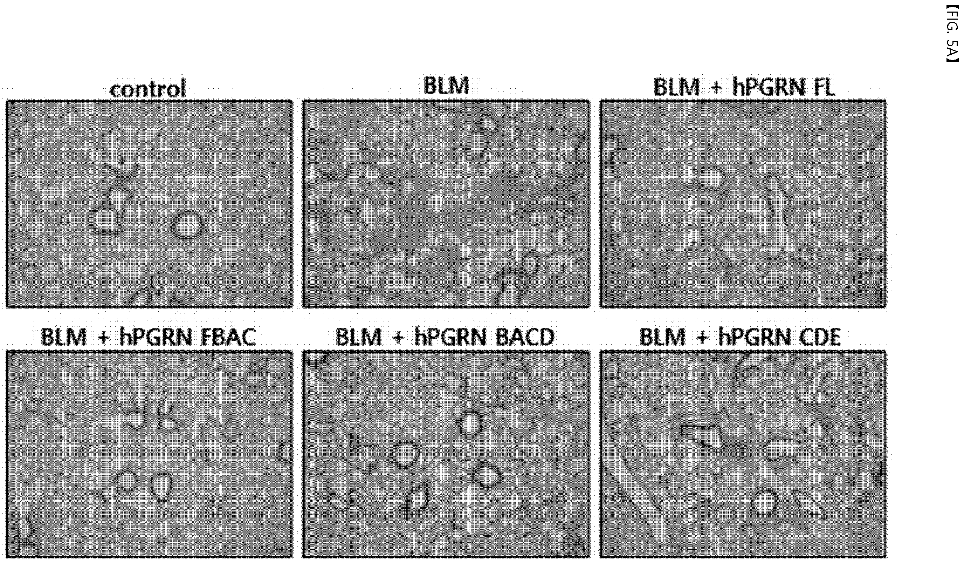

First, as a result of observation of the tissue slides of animal model I (FIG. 5A, in the bleomycin-administered mice, it was observed that, overall, lung tissue is intensively stained, compared to a control, as pulmonary and airway fibrosis had occurred. However, it can be confirmed that, in a model treated with the PGRN protein or a fragment thereof (PGRN-FBAC, PGRN-BACD, or PGRN-CDE), since the staining intensity was reduced, the proliferation of fibroblasts, and the production and secretion of collagen were reduced. This shows that the PGRN protein and fragments thereof effectively inhibited the proliferation of fibroblasts and myofibroblasts and the production of collagen.

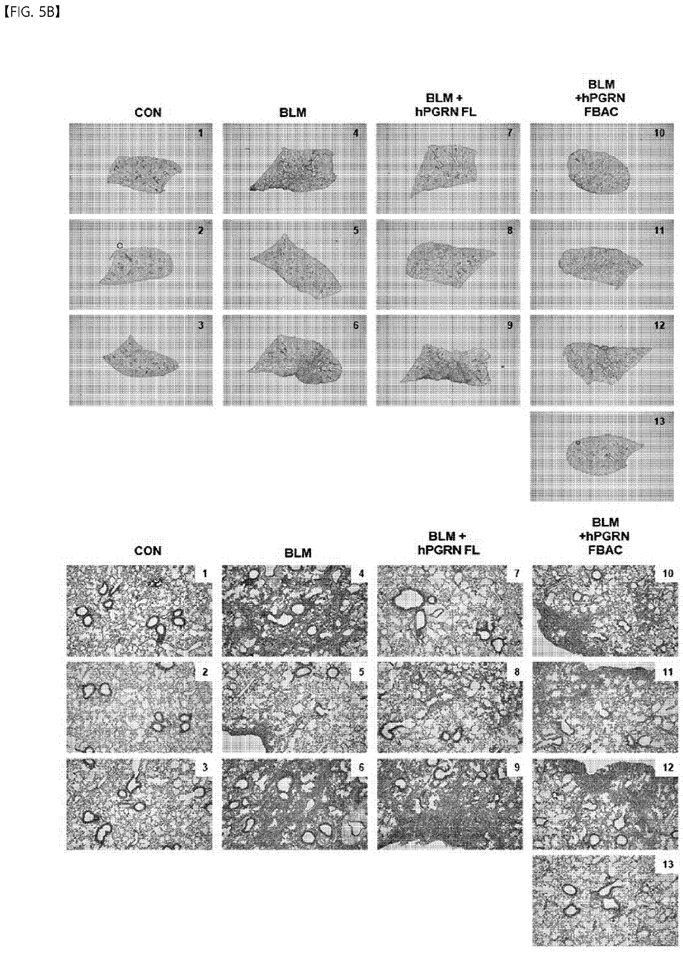

In the case of animal model II (FIG. 5B), the lung tissue of the bleomycin-administered mice was stained with high intensity due to an increase of muscle tissue and fibers due to pulmonary fibrosis, but the staining intensity was remarkably reduced in the mice into which PGRN protein or a fragment thereof (PGRN-FBAC) was injected, confirming that pulmonary and airway fibrosis were improved.

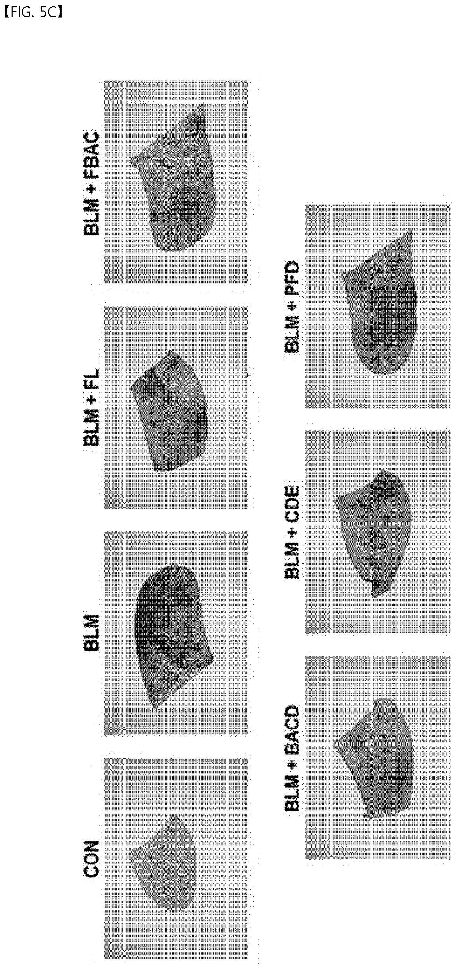

Likewise, in the case of animal model III (FIG. 5C), in the lung tissue of the bleomycin-administered mice, compared to a control, cells and fibers were stained very intensively, but in the mice administered the PGRN protein or a fragment thereof (PGRN-FBAC, PGRN-BACD, or PGRN-CDE), it was seen that the intensity of staining was lowered to a similar or even lower level than that of the mice administered pirfenidone.

The above results show that the PGRN protein according to the present invention and fragments thereof can effectively improve pulmonary and airway fibrosis by inhibiting the proliferation of fibroblasts and myofibroblasts and suppressing collagen production caused thereby.

### Example 6. Confirmation of fibrosis inhibitory effect of PGRN protein and fragment thereof through hydroxyproline measurement

Hydroxyproline is an amino acid, which is a major component of collagen, and is synthesized by post-translational hydroxylation of proline during collagen biosynthesis. Since the level of hydroxyproline in plasma, urine, and body tissue reflects the extent of collagen accumulation, the hydroxyproline level is used to confirm a disease caused by collagen accumulation, specifically, fibrosis. Accordingly, to confirm the therapeutic effects of the PGRN protein and fragments thereof on fibrosis, hydroxyproline levels in lung and airway tissue of a pulmonary fibrosis animal model were measured.

Specifically, after grinding (100 mg/ml, dH₂O) the tissue isolated from the pulmonary fibrosis (animal model II) according to Example 2 and stored at -80 °C, 100 µl of HCl (~12N) was applied to 100 µl of a tissue-ground solution to perform hydrolysis at 120 °C for 3 hours. 10 µl of the hydrolyzed tissue sample was obtained and treated with a chloramine T reagent (Biovision) at room temperature for 5 minutes, and further treated with 100 µl of DMAB (Biovision) at 60 °C for 90 minutes. After the reaction was completed, absorbance was measured at 560 nm using a microplate reader to measure the amount of hydroxyproline in the sample.

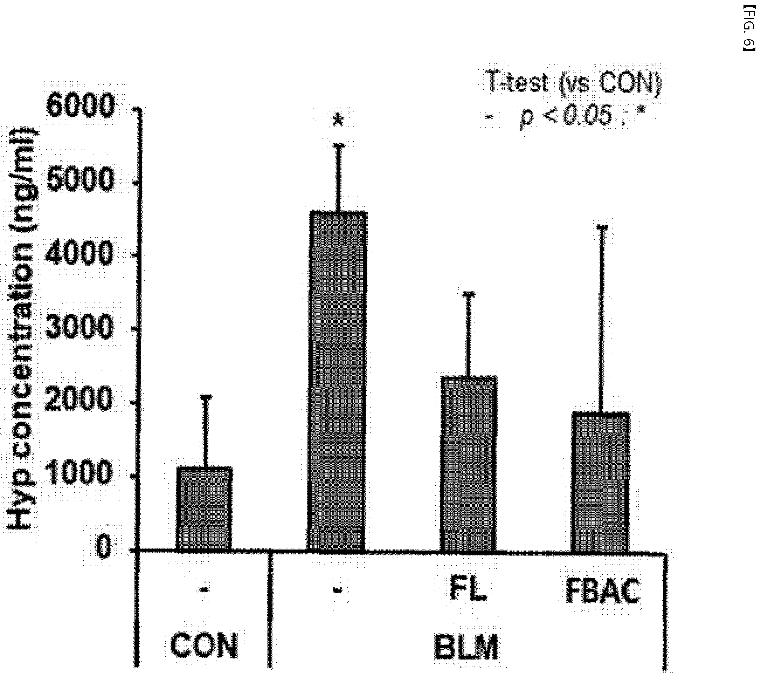

As a result, as shown in FIG. 6, as pulmonary fibrosis progressed in the bleomycin-administered mice, a hydroxyproline level in the lung tissue greatly increased compared to a control. On the other hand, it was seen that the hydroxyproline level in the lung tissue was significantly lowered in the mice into which the PGRN protein or a fragment thereof (PGRN-FBAC) was injected, and particularly, when PGRN-FBAC was treated, lowered 50% or more compared to the group treated with bleomycin alone.

The above results show that the PGRN protein according to the present invention and a fragment thereof can inhibit collagen accumulation, and thus can effectively treat pulmonary and airway fibrosis-related diseases.

### Example 7. Confirmation of weight change in mice by injection of PGRN protein and fragment thereof

Using the pulmonary fibrosis animal model III, weight changes in mice according to injection of the PGRN protein and a fragment thereof were confirmed. Specifically, on days 1, 3, 6, 8, 10, and 13 after the injection of bleomycin into the mice, the PGRN protein or a fragment thereof; or pirfenidone was injected, followed by measurement of the weights of mice.

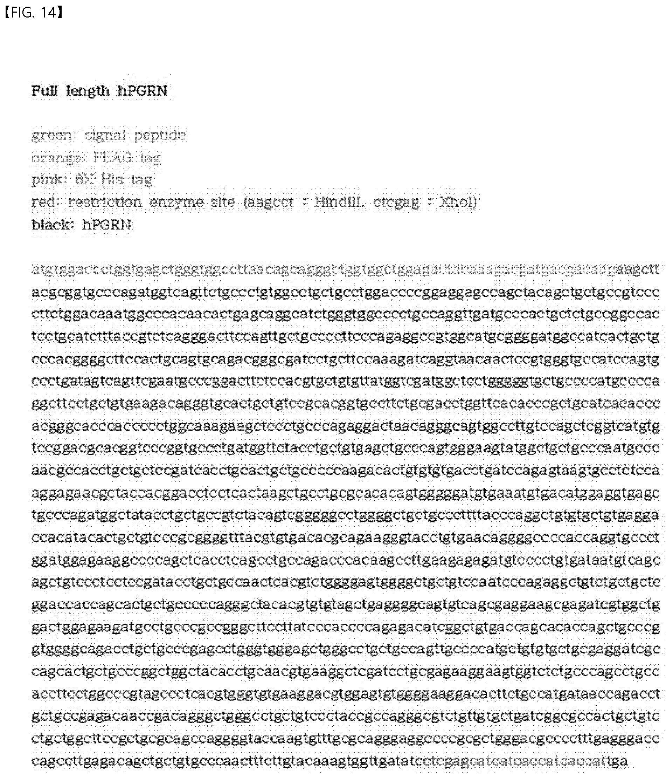

As a result, as shown in FIG.7, 14 days after the injection of bleomycin, it can be confirmed that the mice treated with bleomycin alone tended to have a weight loss of approximately 1 g on average, compared to a control, but the PGRN-BACD-inj ected mice had a relatively decreased weight loss. These results show that, in pulmonary and airway fibrosis diseases, the fragment of the PGRN protein according to the present invention is effective in weight recovery.

### Example 8. Confirmation of changes in fibrosis-related marker and signaling molecule by PGRN protein and fragment thereof

Since the degree of expression of fibronectin, which is one of the representative fibrosis markers, shows a positive correlation with the progression of fibrosis, fibronectin is used to confirm the level of fibrosis. In addition, in the bleomycin-induced pulmonary fibrosis mouse model, the increase in phosphorylation of NFκB by increased TNFα acts as a major cause of pulmonary fibrosis. Accordingly, using a pulmonary epithelial cell line (A549 cells), specific effects of the PGRN protein and a fragment thereof according to the present invention on a fibrosis-related marker and a signaling molecule were confirmed.

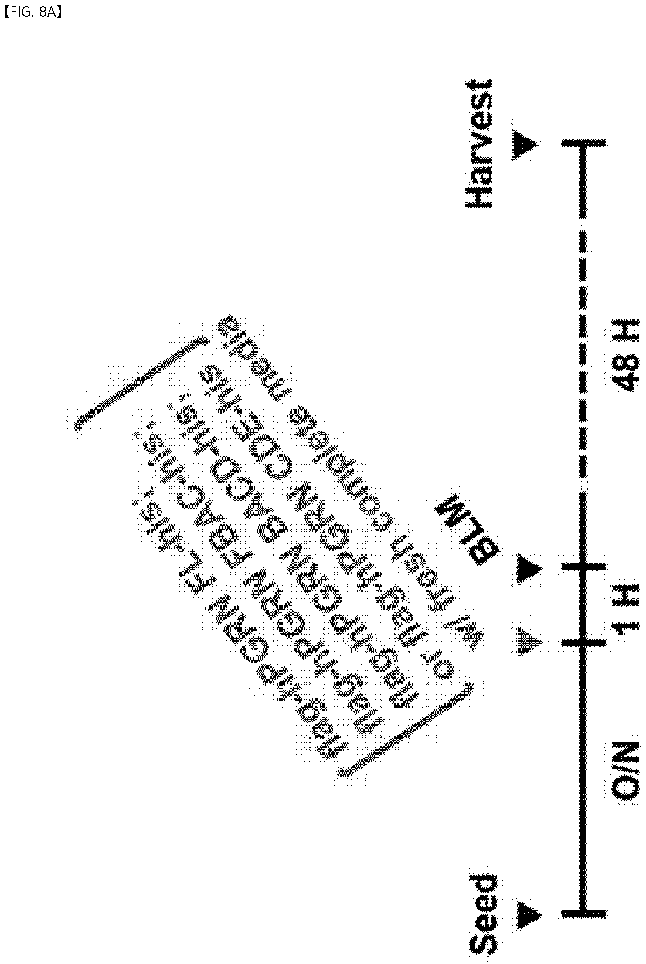

Specifically, as shown in FIG. 8A, the A549 cells were treated with a medium comprising 2 µg/ml of the full-length PGRN protein or a fragment thereof (PGRN-FBAC, PGRN-BACD, or PGRN-CDE) (10% FBS and 1% penicillin/streptomycinsupplemented RPMI 1640) for 1 hour. Afterward, the cells were treated with 50 µg/ml of bleomycin and incubated at 37 °C in an incubator under the condition of 5% CO₂ for 48 hours. The incubated cells were lysed with a RIPA buffer to extract a protein, and the concentration of the extracted protein was quantified by a BCA method. After the electrophoresis of 40 µg of the protein using 8% SDS-PAGE, the protein was transferred to a PVDF membrane. After transfer, the membrane was blocked with 5% skim milk, and reacted on a shaker with primary antibodies overnight (4 °C). Afterward, the membrane was washed twice for 7 minutes each with a TBS-T buffer on a shaker, treated with secondary antibodies, and reacted at room temperature for 1 hour. Subsequently, the membrane was washed twice for 7 minutes each using a TBS-T buffer on a shaker, followed by measurement of chemiluminescence.

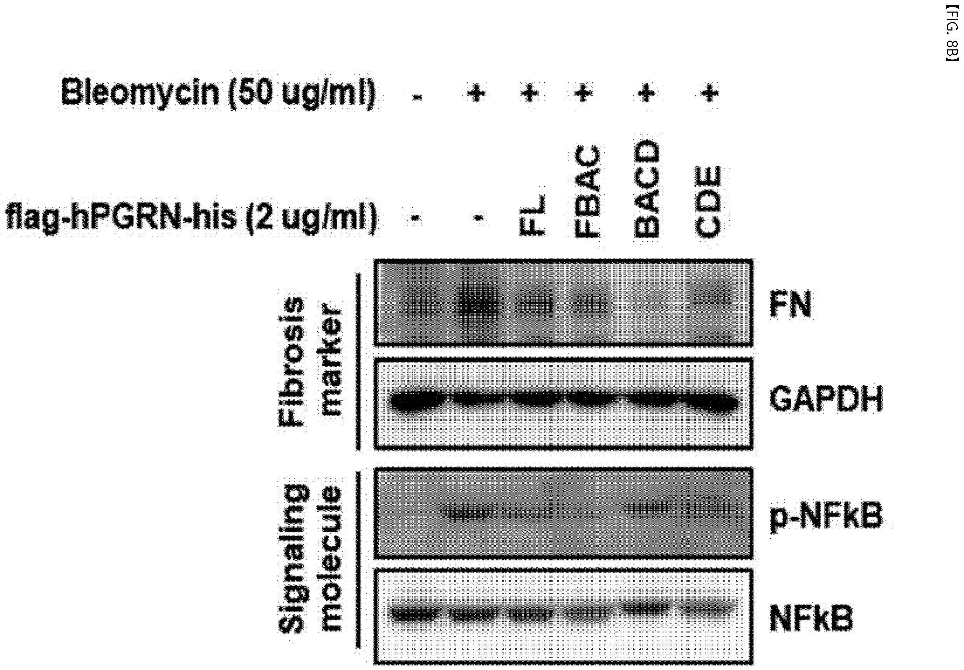

As a result, as shown in FIG. 8B, in cells which were not treated with the PGRN protein and a fragment thereof, the level of the fibrosis marker, fibronectin, was greatly increased by bleomycin compared to a control, and the level of phosphorylated NFκB (p-NFκB) which promotes fibrosis was greatly increased. On the other hand, in the cells treated with the PGRN protein or a fragment thereof, it can be confirmed that, despite the bleomycin treatment, the fibronectin and p-NFκB levels were greatly reduced.

The above results show that the PGRN protein according to the present invention and a fragment thereof suppressed the levels and activities of signaling molecules that mediate fibrosis, thereby effectively inhibiting fibrosis.

### Example 9. Confirmation of change in fibrosis marker by PGRN protein and fragment thereof in pulmonary fibroblast cell line

Since the degrees of the secretion and accumulation of COL1A1, and the expression of F-actin due to fibroblasts are positively correlated with the progression of fibrosis, these factors are used to confirm the degree of fibrosis. Accordingly, after stimulation with TGF-β, pulmonary fibroblasts were treated with the PGRN protein according to the present invention and a fragment thereof, followed by confirming the change in the fibrosis marker COL1A1 through western blotting and ELISA.

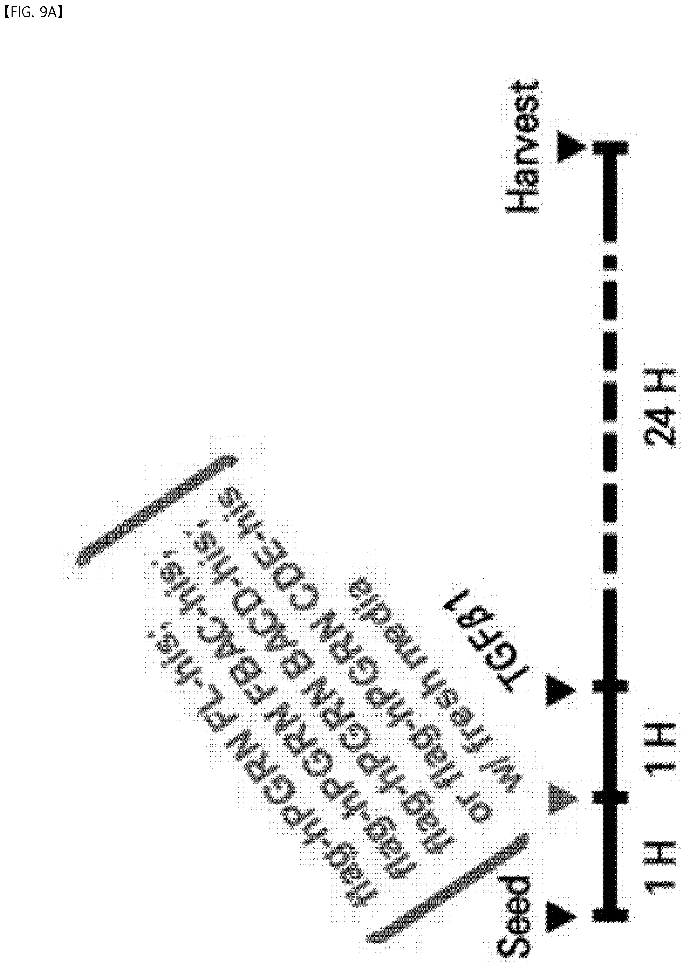

As shown in FIG. 9A, the pulmonary fibroblast cell line (MRC-5 cells) was 3D-cultured in rat tail collagen type I. Specifically, the cells were mixed in a collagen gel (seeding) and solidified in a ϕ35 confocal dish for 1 hour. Afterward, the cells were treated with a serum-deficient MEM medium (1% penicillin/streptomycin) comprising 2 µg/ml of the full-length PGRN protein or a fragment thereof (PGRN-FBAC, PGRN-BACD, or PGRN-CDE) for 1 hour. After an hour, the cells were treated with 10 ng/ml of TGF-β and incubated at 37 °C in an incubator under the condition of 5% CO₂ for 24 hours. After 24 hours, to measure the amount of protein secreted from the cells through western blotting, the culture solution was acquired, 40 µl of the culture solution was subjected to electrophoresis on 8% SDS-PAGE, followed by transfer to a PVDF membrane. After transfer, the membrane was blocked with 5% skim milk, and then overnight reacted with primary antibodies (COL1A1 antibodies) on a shaker (4 °C). Afterward, the membrane was washed twice for 7 minutes each with a TBS-T buffer on a shaker, treated with secondary antibodies, and reacted at room temperature for 1 hour. Subsequently, the membrane was washed twice for 7 minutes each with a TBS-T buffer on a shaker, followed by measurement of chemiluminescence.

In addition, to measure the degree of COL1A1 secretion from pulmonary fibroblasts through ELISA, a 96-well immunoplate was coated overnight at room temperature by treatment with hCOL1A1 capture antibodies. Afterward, the plate was washed with a PBS-T buffer three times, and then blocked for 1 hour. The plate was then washed three times with a PBS-T buffer, and treated with 100 µl of the culture solution at room temperature for 2 hours. Again, the plate was washed with a PBS-T buffer three times, and then reacted with the detected antibodies at room temperature for 2 hours. Subsequently, the plate was washed with a PBS-T buffer three times, and reacted with streptavidin-HRP antibodies at room temperature for 20 minutes. After washing three times with a PBS-T buffer, the plate was treated with a coloring reagent for 20 minutes and treated with a stop buffer, followed by measurement of absorbance at 450 nm. In addition, the collagen gel comprising remaining cells after the culture solution had been removed was washed with PBS three times for 5 minutes, and then fixed with 4% paraformaldehyde at room temperature overnight. Afterward, the resultant was washed with PBS three times, and reacted in 0.2% Triton-X100/PBS at room temperature for 30 minutes. The collagen gel was washed with PBS three times for 10 minutes, and then blocked in 3% BSA/PBS at room temperature for 30 minutes. Subsequently, the collagen gel was washed with PBS three times for 20 minutes, and then stained with 1 U/ml of phalloidin-rhodamine for 1 hour. The stained collagen gel was washed with PBS three times for 10 minutes, and then mounted with a mounting solution comprising DAPI. Afterward, the expression level of F-actin was observed by confocal microscopy.

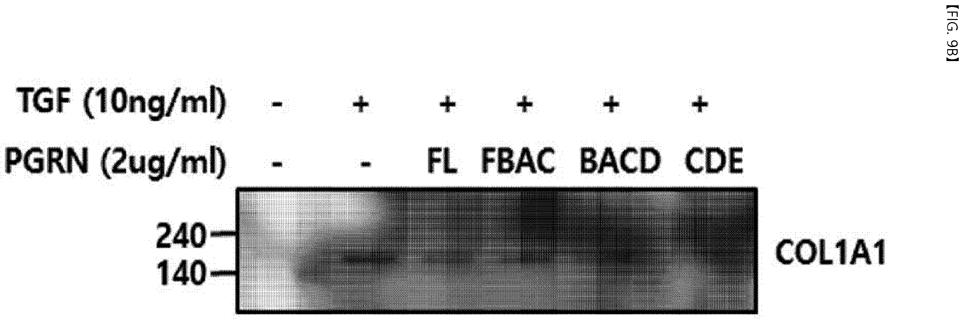

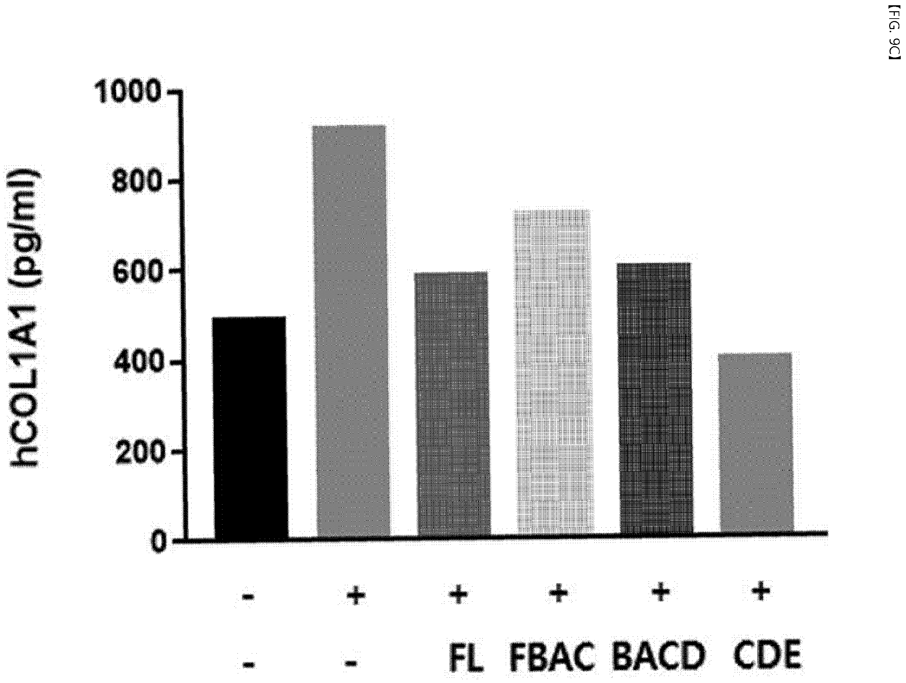

The COL1A1 changes caused by the PGRN protein and a fragment thereof are shown in FIGS. 9B and 9C. In the pulmonary fibroblasts treated with TGF-β alone, compared to a control, it can be confirmed that a COL1A1 level increased, but the cells treated with full-length PGRN, PGRN-FBAC, PGRN-BACD, or PGRN-CDE were greatly decreased in COL1A1 expression (FIG. 9B). Even when the amount of the secreted COL1A1 was quantified by ELISA, it can be confirmed that COL1A1 secretion of the pulmonary fibroblasts was greatly inhibited by the PGRN protein and a fragment thereof (FIG. 9C).

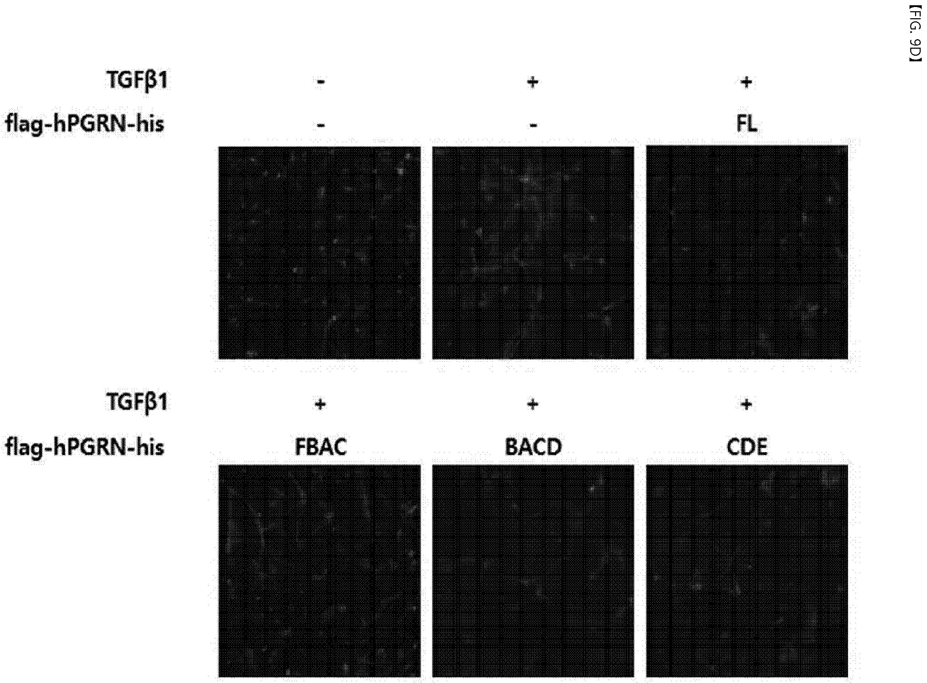

The F-actin changes caused by the PGRN protein and a fragment thereof are shown in FIG. 9D. As a result of confirming the F-actin level in the pulmonary fibroblasts by immunofluorescent staining, it can be confirmed that, in a group treated with TGF-β alone, compared to a negative control, an F-actin level greatly increased, but significantly decreased according to treatment with full-length PGRN, PGRN-FBAC, PGRN-BACD, or PGRN-CDE.

That is, from the above results, it can be seen that, when the main mediator of fibrosis, i.e., fibroblasts, is treated with the PGRN protein according to the present invention and a fragment thereof, the level of the fibrosis marker greatly decreases, supporting the therapeutic effects of the PGRN protein and a fragment thereof on fibrosis.

Summarizing the above examples,, the present inventors confirmed that the PGRN protein and a fragment thereof according to the present invention not only significantly improves inflammation in tissue but also effectively inhibits fibrosis in an animal model in which pulmonary or airway fibrosis had occurred. In addition, through *in vitro* experiments using a pulmonary cell line and fibroblasts, the fibrosis marker level and activity inhibitory effects of the PGRN protein and a fragment thereof were clearly confirmed. Accordingly, the PGRN protein according to the present invention and a fragment thereof are expected to prevent diseases associated with pulmonary and airway fibrosis or be used as therapeutic agents.

It should be understood by those of ordinary skill in the art that the above description of the present invention is exemplary, and the exemplary embodiments disclosed herein can be easily modified into other specific forms without departing from the technical spirit or essential features of the present invention. Therefore, the exemplary embodiments described above should be interpreted as illustrative in all aspects and not restrictive.

### [Industrial Applicability]

The present invention relates to a pharmaceutical composition for preventing or treating pulmonary or airway fibrosis disease, which comprises a PGRN protein or an active fragment thereof as an active ingredient, and was completed by confirming that progranulin (PGRN) and its active fragment can effectively inhibit pulmonary and airway inflammation and fibrosis. Specifically, the present inventors confirmed that, as a result of injecting PGRN and its active fragments (PGRN-FBAC, PGRN-BACD, and PGRN-CDE) into pulmonary fibrosis animal models, the infiltration of inflammatory cells in lung and bronchial tissue and fibroblast proliferation and collagen production are not only reduced, but tissue damage caused by inflammation is also improved. In addition, according to treatment of the pulmonary epithelial cell line and pulmonary fibroblasts with PGRN and its active fragments after the induction of inflammation responses, it was confirmed that the levels and activities of the fibrosis marker fibronectin, p-NFκB, COL1A1, and F-actin are reduced. The above results show that the PGRN protein and its active ingredients can improve pulmonary and airway inflammation and fibrosis and thus can be expected to be used as therapeutic means for various diseases caused by pulmonary and airway fibrosis.

## Claims

1. A pharmaceutical composition for preventing or treating a pulmonary or airway fibrosis disease, comprising a progranulin (PGRN) or an active fragment thereof as an active ingredient.

2. The pharmaceutical composition of claim 1, wherein the active fragment is one or more selected from the group consisting of:
(a) a C granulin domain of the PGRN protein;
(b) a FBAC granulin domain of the PGRN protein;
(c) a BACD granulin domain of the PGRN protein; and
(d) a CDE granulin domain of the PGRN protein.

3. The pharmaceutical composition of claim 1, wherein the PGRN protein or an active fragment thereof comprises the amino acid sequence of any one selected from the group consisting of SEQ ID NOs: 1 to 5.

4. The pharmaceutical composition of claim 1, which inhibits the fibrosis or inflammation of lung or bronchial tissue.

5. The pharmaceutical composition of claim 1, wherein the pulmonary or airway fibrosis disease is one or more selected from the group consisting of pulmonary fibrosis, idiopathic pulmonary fibrosis, idiopathic interstitial pneumonia, chronic obstructive pulmonary disease, asthma, airway fibrosis, chronic bronchitis, acute bronchitis, bronchiolitis, chronic obstructive airway disease, and bronchiolitis obliterans.

6. The pharmaceutical composition of claim 1, which satisfies one or more characteristics selected from the group consisting of
(i) inhibition of tissue infiltration of inflammatory cells;
(ii) reduction in collagen level in tissue; and
(iii) reduction in hydroxyproline level in tissue.

7. The pharmaceutical composition of claim 1, which inhibits the level or activity of one or more selected from the group consisting of fibronectin, phosphorylated NFκB, COL1A1, and F-actin.

8. The pharmaceutical composition of claim 1, which is for treating a patient with one or more mutants selected from the group consisting of fibronectin, NFκB, COL1A1, and F-actin.

9. A pharmaceutical composition for preventing or treating a pulmonary or airway fibrosis disease, comprising a vector comprising a polynucleotide encoding a progranulin (PGRN) protein or an active fragment thereof, or a cell comprising the vector as an active ingredient.

10. The pharmaceutical composition of claim 9, wherein the active fragment is one or more selected from the group consisting of:
(a) a C granulin domain of the PGRN protein;
(b) a FBAC granulin domain of the PGRN protein;
(c) a BACD granulin domain of the PGRN protein; and
(d) a CDE granulin domain of the PGRN protein.

11. The pharmaceutical composition of claim 9, wherein the PGRN protein or an active fragment thereof comprises the amino acid sequence of any one selected from the group consisting of SEQ ID NOs: 1 to 5.

12. The pharmaceutical composition of claim 9, wherein the polynucleotide encoding the PGRN protein or an active fragment thereof comprises the base sequence of any one selected from the group consisting of SEQ ID NOs: 6 to 10.

13. The pharmaceutical composition of claim 9, wherein the vector is linear DNA, plasmid DNA, or a recombinant viral vector.

14. A kit for preventing or treating a pulmonary or airway fibrosis disease, comprising the composition of any one of claims 1 to 13.

15. A method of preventing or treating a pulmonary or airway fibrosis disease, comprising:
administering a progranulin (PGRN) protein or an active fragment thereof; a vector comprising a polynucleotide encoding the PGRN protein or active fragment thereof; and/or cells comprising the vector into a subject in need thereof.

16. A use of a progranulin (PGRN) protein or an active fragment thereof; a vector comprising a polynucleotide encoding the PGRN protein or active fragment thereof; and/or cells comprising the vector to prevent or treat a pulmonary or airway fibrosis disease.

17. A use of a progranulin (PGRN) protein or an active fragment thereof; a vector comprising a polynucleotide encoding the PGRN protein or active fragment thereof; and/or cells comprising the vector to prepare a drug for treating a pulmonary or airway fibrosis disease.
